(19) Europäisches Patentamt / European Patent Office / Office européen des brevets

(11) **EP 3 542 633 A1**

(12) **EUROPEAN PATENT APPLICATION**

(43) Date of publication:
**25.09.2019 Bulletin 2019/39**

(21) Application number: **19168718.5**

(22) Date of filing: **06.03.2015**

(51) Int Cl.:
*A23C 9/12* (2006.01)   *A23C 9/13* (2006.01)
*A23C 9/18* (2006.01)   *A23C 21/02* (2006.01)
*C12P 19/04* (2006.01)   *C12N 9/10* (2006.01)
*C12N 9/38* (2006.01)   *A23C 9/16* (2006.01)
*C12P 19/14* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.03.2014 EP 14158189**

(62) Document number(s) of the earlier application(s) in accordance with Art. 76 EPC:
**15708221.5 / 3 113 622**

(71) Applicant: **Arla Foods amba**
**8260 Viby J (DK)**

(72) Inventors:
• RAY, Colin A.
  **17237 Stockholm (SE)**
• OSMAN, Ali
  **8471 Sabro (DK)**

(74) Representative: **Guardian**
**IP Consulting I/S**
**Diplomvej, Building 381**
**2800 Kgs. Lyngby (DK)**

Remarks:
This application was filed on 11-04-2019 as a divisional application to the application mentioned under INID code 62.

(54) **LACTOSE-REDUCED MILK PRODUCTS CONTAINING GALACTO-OLIGOSACCHARIDES AND LIMITED AMOUNT OF MONOSACCHARIDES AND A METHOD OF PRODUCTION**

(57) The present invention relates to liquid milk products having a pH of at least 5.0 comprising
- at least 0.9% (w/w) GOS,
- at most 1.5% (w/w) lactose,
- a total content of monosaccharide of at most 3.0% (w/w), and
- a weight ratio between glucose and galactose of least 2: 1
and powdered compositions obtained therefrom.
Preferably, the compositions comprise a low amount of furosine.

Fig. 1

SEQUENCE LISTING

<110> Arla Foods amba

<120> LACTOSE-REDUCED MILK PRODUCTS CONTAINING GALACTO-OLIGOSACCHARIDES AND MONOSACCHARIDES AND A METHOD OF PRODUCTION

<130> P1635PC00

<160> 1

<170> BiSSAP 1.0

<210> 1

<211> 1720
<212> PRT
<213> Bifidobacterium bifidum

<220>
<221> SOURCE
<222> 1..1720
<223> /mol_type="protein"
        /organism="Bifidobacterium bifidum"

<400> 1
Val Glu Asp Ala Thr Arg Ser Asp Ser Thr Thr Gln Met Ser Ser Thr
1               5                   10                  15
Pro Glu Val Val Tyr Ser Ser Ala Val Asp Ser Lys Gln Asn Arg Thr
            20                  25                  30
Ser Asp Phe Asp Ala Asn Trp Lys Phe Met Leu Ser Asp Ser Val Gln
            35                  40                  45
Ala Gln Asp Pro Ala Phe Asp Asp Ser Ala Trp Gln Gln Val Asp Leu
        50                  55                  60
Pro His Asp Tyr Ser Ile Thr Gln Lys Tyr Ser Gln Ser Asn Glu Ala
65                  70                  75                  80
Glu Ser Ala Tyr Leu Pro Gly Gly Thr Gly Trp Tyr Arg Lys Ser Phe
                85                  90                  95
Thr Ile Asp Arg Asp Leu Ala Gly Lys Arg Ile Ala Ile Asn Phe Asp
            100                 105                 110
Gly Val Tyr Met Asn Ala Thr Val Trp Phe Asn Gly Val Lys Leu Gly
            115                 120                 125
Thr His Pro Tyr Gly Tyr Ser Pro Phe Ser Phe Asp Leu Thr Gly Asn
            130                 135                 140
Ala Lys Phe Gly Gly Glu Asn Thr Ile Val Val Lys Val Glu Asn Arg
145                 150                 155                 160
Leu Pro Ser Ser Arg Trp Tyr Ser Gly Ser Gly Ile Tyr Arg Asp Val
                165                 170                 175
Thr Leu Thr Val Thr Asp Gly Val His Val Gly Asn Asn Gly Val Ala
            180                 185                 190
Ile Lys Thr Pro Ser Leu Ala Thr Gln Asn Gly Gly Asp Val Thr Met
            195                 200                 205
Asn Leu Thr Thr Lys Val Ala Asn Asp Thr Glu Ala Ala Ala Asn Ile
        210                 215                 220
Thr Leu Lys Gln Thr Val Phe Pro Lys Gly Gly Lys Thr Asp Ala Ala
225                 230                 235                 240
Ile Gly Thr Val Thr Thr Ala Ser Lys Ser Ile Ala Ala Gly Ala Ser
                245                 250                 255
Ala Asp Val Thr Ser Thr Ile Thr Ala Ala Ser Pro Lys Leu Trp Ser
            260                 265                 270
Ile Lys Asn Pro Asn Leu Tyr Thr Val Arg Thr Glu Val Leu Asn Gly
            275                 280                 285
Gly Lys Val Leu Asp Thr Tyr Asp Thr Glu Tyr Gly Phe Arg Trp Thr

```
                290                         295                         300
Gly Phe Asp Ala Thr Ser Gly Phe Ser Leu Asn Gly Glu Lys Val Lys
305                     310                         315                     320
Leu Lys Gly Val Ser Met His His Asp Gln Gly Ser Leu Gly Ala Val
                325                         330                         335
Ala Asn Arg Arg Ala Ile Glu Arg Gln Val Glu Ile Leu Gln Lys Met
                340                         345                         350
Gly Val Asn Ser Ile Arg Thr Thr His Asn Pro Ala Ala Lys Ala Leu
                355                         360                         365
Ile Asp Val Cys Asn Glu Lys Gly Val Leu Val Val Glu Glu Val Phe
370                         375                         380
Asp Met Trp Asn Arg Ser Lys Asn Gly Asn Thr Glu Asp Tyr Gly Lys
385                         390                         395                     400
Trp Phe Gly Gln Ala Ile Ala Gly Asp Asn Ala Val Leu Gly Gly Asp
                405                         410                         415
Lys Asp Glu Thr Trp Ala Lys Phe Asp Leu Thr Ser Thr Ile Asn Arg
                420                         425                         430
Asp Arg Asn Ala Pro Ser Val Ile Met Trp Ser Leu Gly Asn Glu Met
                435                         440                         445
Met Glu Gly Ile Ser Gly Ser Val Ser Gly Phe Pro Ala Thr Ser Ala
450                         455                         460
Lys Leu Val Ala Trp Thr Lys Ala Ala Asp Ser Thr Arg Pro Met Thr
465                     470                         475                     480
Tyr Gly Asp Asn Lys Ile Lys Ala Asn Trp Asn Glu Ser Asn Thr Met
                485                         490                         495
Gly Asp Asn Leu Thr Ala Asn Gly Gly Val Val Gly Thr Asn Tyr Ser
                500                         505                         510
Asp Gly Ala Asn Tyr Asp Lys Ile Arg Thr Thr His Pro Ser Trp Ala
                515                         520                         525
Ile Tyr Gly Ser Glu Thr Ala Ser Ala Ile Asn Ser Arg Gly Ile Tyr
530                         535                         540
Asn Arg Thr Thr Gly Gly Ala Gln Ser Ser Asp Lys Gln Leu Thr Ser
545                         550                         555                     560
Tyr Asp Asn Ser Ala Val Gly Trp Gly Ala Val Ala Ser Ser Ala Trp
                565                         570                         575
Tyr Asp Val Val Gln Arg Asp Phe Val Ala Gly Thr Tyr Val Trp Thr
                580                         585                         590
Gly Phe Asp Tyr Leu Gly Glu Pro Thr Pro Trp Asn Gly Thr Gly Ser
                595                         600                         605
Gly Ala Val Gly Ser Trp Pro Ser Pro Lys Asn Ser Tyr Phe Gly Ile
610                         615                         620
Val Asp Thr Ala Gly Phe Pro Lys Asp Thr Tyr Tyr Phe Tyr Gln Ser
625                     630                         635                     640
Gln Trp Asn Asp Asp Val His Thr Leu His Ile Leu Pro Ala Trp Asn
                645                         650                         655
Glu Asn Val Val Ala Lys Gly Ser Gly Asn Asn Val Pro Val Val Val
                660                         665                         670
Tyr Thr Asp Ala Ala Lys Val Lys Leu Tyr Phe Thr Pro Lys Gly Ser
                675                         680                         685
Thr Glu Lys Arg Leu Ile Gly Glu Lys Ser Phe Thr Lys Lys Thr Thr
690                         695                         700
Ala Ala Gly Tyr Thr Tyr Gln Val Tyr Glu Gly Ser Asp Lys Asp Ser
705                     710                         715                     720
Thr Ala His Lys Asn Met Tyr Leu Thr Trp Asn Val Pro Trp Ala Glu
                725                         730                         735
Gly Thr Ile Ser Ala Glu Ala Tyr Asp Glu Asn Asn Arg Leu Ile Pro
                740                         745                         750
Glu Gly Ser Thr Glu Gly Asn Ala Ser Val Thr Thr Gly Lys Ala
                755                         760                         765
Ala Lys Leu Lys Ala Asp Ala Asp Arg Lys Thr Ile Thr Ala Asp Gly
770                         775                         780
Lys Asp Leu Ser Tyr Ile Glu Val Asp Val Thr Asp Ala Asn Gly His
785                         790                         795                     800
```

24

```
Ile Val Pro Asp Ala Ala Asn Arg Val Thr Phe Asp Val Lys Gly Ala
             805                 810                 815
Gly Lys Leu Val Gly Val Asp Asn Gly Ser Ser Pro Asp His Asp Ser
             820                 825                 830
Tyr Gln Ala Asp Asn Arg Lys Ala Phe Ser Gly Lys Val Leu Ala Ile
             835                 840                 845
Val Gln Ser Thr Lys Glu Ala Gly Glu Ile Thr Val Thr Ala Lys Ala
         850                 855                 860
Asp Gly Leu Gln Ser Ser Thr Val Lys Ile Ala Thr Thr Ala Val Pro
865                 870                 875                 880
Gly Thr Ser Thr Glu Lys Thr Val Arg Ser Phe Tyr Tyr Ser Arg Asn
             885                 890                 895
Tyr Tyr Val Lys Thr Gly Asn Lys Pro Ile Leu Pro Ser Asp Val Glu
             900                 905                 910
Val Arg Tyr Ser Asp Gly Thr Ser Asp Arg Gln Asn Val Thr Trp Asp
             915                 920                 925
Ala Val Ser Asp Asp Gln Ile Ala Lys Ala Gly Ser Phe Ser Val Ala
         930                 935                 940
Gly Thr Val Ala Gly Gln Lys Ile Ser Val Arg Val Thr Met Ile Asp
945                 950                 955                 960
Glu Ile Gly Ala Leu Leu Asn Tyr Ser Ala Ser Thr Pro Val Gly Thr
             965                 970                 975
Pro Ala Val Leu Pro Gly Ser Arg Pro Ala Val Leu Pro Asp Gly Thr
             980                 985                 990
Val Thr Ser Ala Asn Phe Ala Val His Trp Thr Lys Pro Ala Asp Thr
         995                 1000                1005
Val Tyr Asn Thr Ala Gly Thr Val Lys Val Pro Gly Thr Ala Thr Val
             1010                1015                1020
Phe Gly Lys Glu Phe Lys Val Thr Ala Thr Ile Arg Val Gln Arg Ser
1025                1030                1035                1040
Gln Val Thr Ile Gly Ser Ser Val Ser Gly Asn Ala Leu Arg Leu Thr
             1045                1050                1055
Gln Asn Ile Pro Ala Asp Lys Gln Ser Asp Thr Leu Asp Ala Ile Lys
             1060                1065                1070
Asp Gly Ser Thr Thr Val Asp Ala Asn Thr Gly Gly Gly Ala Asn Pro
             1075                1080                1085
Ser Ala Trp Thr Asn Trp Ala Tyr Ser Lys Ala Gly His Asn Thr Ala
             1090                1095                1100
Glu Ile Thr Phe Glu Tyr Ala Thr Glu Gln Gln Leu Gly Gln Ile Val
1105                1110                1115                1120
Met Tyr Phe Phe Arg Asp Ser Asn Ala Val Arg Phe Pro Asp Ala Gly
             1125                1130                1135
Lys Thr Lys Ile Gln Ile Ser Ala Asp Gly Lys Asn Trp Thr Asp Leu
             1140                1145                1150
Ala Ala Thr Glu Thr Ile Ala Ala Gln Glu Ser Ser Asp Arg Val Lys
         1155                1160                1165
Pro Tyr Thr Tyr Asp Phe Ala Pro Val Gly Ala Thr Phe Val Lys Val
         1170                1175                1180
Thr Val Thr Asn Ala Asp Thr Thr Thr Pro Ser Gly Val Val Cys Ala
1185                1190                1195                1200
Gly Leu Thr Glu Ile Glu Leu Lys Thr Ala Thr Ser Lys Phe Val Thr
             1205                1210                1215
Asn Thr Ser Ala Ala Leu Ser Ser Leu Thr Val Asn Gly Thr Lys Val
         1220                1225                1230
Ser Asp Ser Val Leu Ala Ala Gly Ser Tyr Asn Thr Pro Ala Ile Ile
         1235                1240                1245
Ala Asp Val Lys Ala Glu Gly Glu Gly Asn Ala Ser Val Thr Val Leu
         1250                1255                1260
Pro Ala His Asp Asn Val Ile Arg Val Ile Thr Glu Ser Glu Asp His
1265                1270                1275                1280
Val Thr Arg Lys Thr Phe Thr Ile Asn Leu Gly Thr Glu Gln Glu Phe
             1285                1290                1295
Pro Ala Asp Ser Asp Glu Arg Asp Tyr Pro Ala Ala Asp Met Thr Val
```

```
                  1300                  1305                  1310
      Thr Val Gly Ser Glu Gln Thr Ser Gly Thr Ala Thr Glu Gly Pro Lys
              1315                  1320                  1325
      Lys Phe Ala Val Asp Gly Asn Thr Ser Thr Tyr Trp His Ser Asn Trp
              1330                  1335                  1340
      Thr Pro Thr Thr Val Asn Asp Leu Trp Ile Ala Phe Glu Leu Gln Lys
      1345                  1350                  1355                  1360
      Pro Thr Lys Leu Asp Ala Leu Arg Tyr Leu Pro Arg Pro Ala Gly Ser
              1365                  1370                  1375
      Lys Asn Gly Ser Val Thr Glu Tyr Lys Val Gln Val Ser Asp Asp Gly
              1380                  1385                  1390
      Thr Asn Trp Thr Asp Ala Gly Ser Gly Thr Trp Thr Thr Asp Tyr Gly
              1395                  1400                  1405
      Trp Lys Leu Ala Glu Phe Asn Gln Pro Val Thr Thr Lys His Val Arg
              1410                  1415                  1420
      Leu Lys Ala Val His Thr Tyr Ala Asp Ser Gly Asn Asp Lys Phe Met
      1425                  1430                  1435                  1440
      Ser Ala Ser Glu Ile Arg Leu Arg Lys Ala Val Asp Thr Thr Asp Ile
              1445                  1450                  1455
      Ser Gly Ala Thr Val Thr Val Pro Ala Lys Leu Thr Val Asp Arg Val
              1460                  1465                  1470
      Asp Ala Asp His Pro Ala Thr Phe Ala Thr Lys Asp Val Thr Val Thr
              1475                  1480                  1485
      Leu Gly Asp Ala Thr Leu Arg Tyr Gly Val Asp Tyr Leu Leu Asp Tyr
              1490                  1495                  1500
      Ala Gly Asn Thr Ala Val Gly Lys Ala Thr Val Thr Val Arg Gly Ile
      1505                  1510                  1515                  1520
      Asp Lys Tyr Ser Gly Thr Val Ala Lys Thr Phe Thr Ile Glu Leu Lys
              1525                  1530                  1535
      Asn Ala Pro Ala Pro Glu Pro Thr Leu Thr Ser Val Ser Val Lys Thr
              1540                  1545                  1550
      Lys Pro Ser Lys Leu Thr Tyr Val Val Gly Asp Ala Phe Asp Pro Ala
              1555                  1560                  1565
      Gly Leu Val Leu Gln His Asp Arg Gln Ala Asp Arg Pro Pro Gln Pro
              1570                  1575                  1580
      Leu Val Gly Glu Gln Ala Asp Glu Arg Gly Leu Thr Cys Gly Thr Arg
      1585                  1590                  1595                  1600
      Cys Asp Arg Val Glu Gln Leu Arg Lys His Glu Asn Arg Glu Ala His
              1605                  1610                  1615
      Arg Thr Gly Leu Asp His Leu Glu Phe Val Gly Ala Ala Asp Gly Ala
              1620                  1625                  1630
      Val Gly Glu Gln Ala Thr Phe Lys Val His Val His Ala Asp Gln Gly
              1635                  1640                  1645
      Asp Gly Arg His Asp Asp Ala Asp Glu Arg Asp Ile Asp Pro His Val
              1650                  1655                  1660
      Pro Val Asp His Ala Val Gly Glu Leu Ala Arg Ala Ala Cys His His
      1665                  1670                  1675                  1680
      Val Ile Gly Leu Arg Val Asp Thr His Arg Leu Lys Ala Ser Gly Phe
              1685                  1690                  1695
      Gln Ile Pro Ala Asp Asp Met Ala Glu Ile Asp Arg Ile Thr Gly Phe
              1700                  1705                  1710
      His Arg Phe Glu Arg His Val Gly
              1715                  1720
```

**Description**

**FIELD OF THE INVENTION**

The present invention relates to a method of producing milk products containing galacto-oligosaccharides (GOS) at low temperature using beta-galactosidase enzymes having a high level of transgalactosylation activity. The present invention furthermore relates to the obtained milk products containing GOS and monosaccharide sweeteners characterised in a weight ratio between glucose and galactose of at least 2:1.

**BACKGROUND**

Galacto-oligosaccharide (GOS) has for long been recognised as a health promoting agent which acts as growth factor for a number of beneficial bacterial strains within the gastrointestinal system of mammals. The ingestion of GOS has been associated with a reduced risk of developing diarrhoea and other gastro-intestinal infections, as well as a strengthened immune system. See for example Macfarlane et al (Aliment Pharmacol Ther 24, 701-714).
GOS has been used as a functional food ingredient in infant formula products and has been suggested used in a number of different food applications.
Initial attempts to produce GOS directly in skimmed milk have previously been reported in Rodrigues-Colinas 2012 (Rodrigues-Colinas et al.; J. Agric. Food Chem. 2012, 60, 6391-6398), but only with a maximum yield of GOS of approx. 0.7% (w/w) corresponding to approx. 15% of the total carbohydrates.
A similar approach was described in Rodrigues-Colinas 2014 (Rodrigues-Colinas et al.; Food Chemistry 145 (2014), 388-394) using two different incubation temperatures (4 degrees C and 40 degrees C). A GOS content of only 0.8% (w/w) including traces of GOS-DP4 was obtained.
Synthesis of GOS in milk and yoghurt is also suggested in WO 2013/182,686. However, WO 2013/182,686 neither provides specific details regarding the chemical composition of such GOS-containing milk nor the use of low temperature during the synthesis.

**SUMMARY OF THE INVENTION**

The present inventors have been particularly interested in GOS-containing milk products which are useful for the preparation of long shelf life products, such as long shelf life liquid milk products or long shelf life powdered milk products.
The present inventors have discovered that liquid milk products having a GOS-content of at least 0.9% (w/w), a monosaccharide content of at most 3.0% (w/w), and having a weight ratio between glucose and galactose of at least 2:1 are particularly suitable for the preparation of long shelf-life products and have seen indications that such products have improved stability and sensory properties.
Thus, an aspect of the invention pertains to a liquid milk product having a pH of at least 5.0, the liquid milk product comprising:

- at least 0.9% (w/w) GOS,
- at most 3% (w/w) lactose, and
- a weight ratio between glucose and galactose of at least 2:1.

For example, the liquid milk product may have a total content of monosaccharide of at most 3.0% (w/w).
In the context of the present invention the terms "galacto-oligosaccharide" or "GOS" pertain to oligosaccharides having the stoichiometric formula $(Gal)_i Glc$ or $(Gal)_j$, where i=1-8 and j=2-9. GOS is typically present as a mixture of various GOS molecules having varying degrees of polymerisation and different linkage structures. The mixture may include both linear GOS molecules and branched GOS molecules. Lactose is not counted as a GOS molecule.

**BRIEF DESCRIPTION OF THE FIGURES**

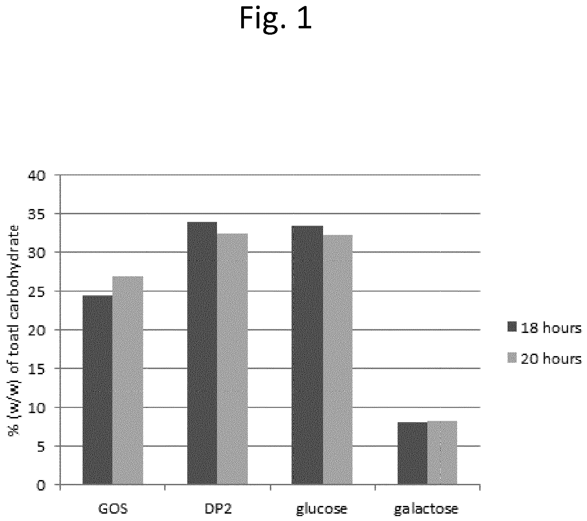

Figure 1 shows the distribution of carbohydrates in a skimmed milk sample which has been treated with the BIF917 enzyme at 8 degrees C for 24 hours.

**DETAILED DESCRIPTION OF THE INVENTION**

A broad aspect of the invention pertains to a liquid milk product having a pH of at least 5.0, the liquid milk product comprising:

- at least 0.9% (w/w) GOS,
- at most 3% (w/w) lactose.

For example, the liquid milk product may furthermore contain a total amount of glucose and galactose of at least 0.1% (w/w).

The liquid milk product may furthermore have a weight ratio between glucose and galactose of at least 2:1.

The present inventors have found that the above liquid milk product provides an improved sweetness per digestible carbohydrate calorie of the milk product.

In some preferred embodiments of the invention the liquid milk product has a total content of monosaccharide of at most 3.0% (w/w).

The liquid milk product may e.g. be obtainable by the method described herein.

Sometimes it is furthermore preferred that the liquid milk product has not been heated to temperatures above 10 degrees C for more than 30 minutes during its entire processing and preferably not for more than 15 minutes.

In some preferred embodiments of the invention, the liquid milk product has a content of lipopolysaccharides of at most 5100 EU lipopolysaccharides (LPS) per liter, and preferably even less. For example, the liquid milk product may have a content of lipopolysaccharides of at most 1000 EU LPS per liter. The liquid milk product may e.g. have a content of lipopolysaccharides of at most 200 EU LPS per liter.

The outer membrane of Gram negative bacteria, in contrast to Gram positive bacteria, contains lipopolysaccharides (LPS) which are endotoxins. A single bacterial cell contains approximately $3.5 \times 10^6$ LPS molecules. LPS is a complex, negatively charged molecule composed of a distal polysaccharide chain called the O-specific chain, a core polysaccharide and a lipid moiety referred to as lipid A. LPS acts as endotoxin resulting in induction of strong inflammatory responses in animals and human beings and is involved in the development of several diseases, e.g. sepsis. The toxic part of LPS is the lipid A moiety which consists of two phosphorylated glucosamine residues and at least 6 fatty acids. These two phosphate groups are essential for the bioactivity of LPS. Incorporated in the outer leaflet of the Gram-negative bacterial cell membrane, the LPS molecule is relatively non-toxic as the lipid A moiety is more or less stowed away. However, when a dividing or dying bacterium spontaneously releases LPS into the mammalian circulation, it can interact with several proteins such as albumin, lactoferrin, high-density lipoproteins (HDL), low-density lipoprotein (LDL) and bacterial permeability-increasing protein (BPI). The LPS-binding protein (LBP) is the most important protein in this respect. The LBP-LPS complex binds to CD 14 ('Cluster of differentiation 14') after being released from the membrane of the cells of the myeloid lineage (macrophages, monocytes and polymorphonuclear leukocytes). The CD 14 requires an accessory receptor complex, the toll-like receptor 4/myeloid differentiation-2 complex (TLR4/MD-2) to initiate a cell signal transduction cascade in the cell. This cascade promotes nuclear translocation of NF-KB and transcription of pro-inflammatory cytokines such as Tumor Necrosis Factor alpha (TNFcc). TNFcc and other pro-inflammatory cytokines induce vascular permeability, enhanced blood flow and neutrophil recruitment to the LPS source as well as systemic responses such as fever. At high concentrations, LPS becomes toxic by overstimulation of TLR4 signalling, leading to an excessive inflammatory response that results in adverse reactions such as septic shock. An aberrant immune response to LPS or other bacterial antigens (e.g. flagellin or T cell epitopes) has been linked to inflammatory bowel disease (IBD) and necrotizing enterocolitis (NEC). NEC predominantly affects premature infants and is assumed to be caused by an immature immune response to commensal organisms that colonize the gut after birth.

Premature infants probably have impaired LPS sensing since they miss an essential molecule from the TLR4-induced signalling pathway, and neonates are therefore predisposed to NEC upon microbial colonization of the immature intestine since one of the most abundant sources of LPS encountered by vertebrates is their resident gut microbiota. These gut microbiota do not elicit pathological inflammation in adult hosts. There are also indications that LPS plays an important role in the induction of systemic inflammation. Since new-born babies have a higher permeability of their gut epithelium during the first months or their lives, pathogenic substances have a higher chance of crossing the epithelial barrier and inducing inflammation in the child.

Raw milk can contain significant numbers of Gram negative bacteria. For health safety reasons, in food, bacteria, including the Gram negative bacteria, are killed by pasteurisation and sterilisation techniques. Unfortunately, killing the Gram negative bacteria does not result in complete degradation of the membrane of Gram negative bacteria and the LPS molecules stay largely intact and are therefore released into the composition. In addition, during the processing of a food composition, LPS is released from the bacterial wall due to the shearing forces caused by the food processing techniques. LPS is heat stable at 100°C and can survive during the processing of food products.

In some preferred embodiments of the invention the liquid milk product contains inactivated beta-galactosidase enzyme which has been used for in-milk GOS production. The inactivated beta-galactosidase enzyme may e.g. be thermally inactivated beta-galactosidase enzyme. In the context of the present invention the term "inactivated enzyme" means that the enzyme has been denatured, modified or degraded and has lost its beta-galactosidase activity.

In some embodiments of the invention the liquid milk product has a total content of carbohydrate of at most 15% (w/w). The liquid milk product may have a total content of carbohydrate of at most 10% (w/w). The liquid milk product may e.g. have a total content of carbohydrate of at most 5% (w/w). Alternatively, the liquid milk product may have a total content of carbohydrate of at most 3% (w/w). This may e.g. be the case in liquid milk products where the initial lactose concentration has been reduced by about 30-35 % prior to applying the enzyme.

In the context of the present invention the term "carbohydrate" encompasses both small carbohydrates, such as mono-saccharides and di-saccharides, and larger saccharides, such as polysaccharides and oligosaccharides.

In the context of the present invention, when a composition is said to comprise, contain or have X % (w/w) of a specified component, the weight percentage of the specified component is calculated relative to the total weight of the composition unless it is stated otherwise.

The liquid milk product may e.g. have a total content of carbohydrate in the range of 1-15% (w/w). For example, the liquid milk product may have a total content of carbohydrate in the range of 2-10% (w/w), such as in the range of 3-8% (w/w). Alternatively, the liquid milk product may have a total content of carbohydrate in the range of 4-6% (w/w), such as approx. 5% (w/w).

In some low carbohydrate embodiments of the invention, it is preferred that the liquid milk product has a total content of carbohydrate in the range of 1-4% (w/w), such as in the range of 2-3% (w/w).

In some embodiments of the invention the liquid milk product has a total content of mono- and disaccharides of at most 10% (w/w). The liquid milk product may have a total content of mono- and disaccharides of at most 8% (w/w). The liquid milk product may e.g. have a total content of mono- and disaccharides of at most 5% (w/w), such as at most 3% (w/w). Alternatively, the liquid milk product may have a total content of mono- and disaccharides of at most 2% (w/w), such as at most 1% (w/w).

The liquid milk product may e.g. have a total content of mono- and disaccharides in the range of 0.1-10% (w/w). For example, the liquid milk product may have a total content of mono- and disaccharides in the range of 1-8% (w/w), such as in the range of 1.5-6% (w/w). Alternatively, the liquid milk product may have a total content of mono- and disaccharides in the range of 2-4% (w/w), such as approx. 3% (w/w).

In some preferred embodiments of the invention the liquid milk product has a total content of lactose of at most 3% (w/w). The liquid milk product may have a total content of lactose of at most 2.5% (w/w). The liquid milk product may e.g. have a total content of lactose of at most 2.0% (w/w). Alternatively, the liquid milk product may have a total content of lactose of at most 1.5% (w/w).

The liquid milk product may e.g. have a total content of lactose of at most 1.0% (w/w), such as at most 0.5% (w/w). Alternatively, the liquid milk product may have a total content of lactose of at most 0.2% (w/w), such as at most 0.1% (w/w).

The liquid milk product may e.g. have a total content of lactose in the range of 0.05-3% (w/w). For example, the liquid milk product may have a total content of lactose in the range of 0.1-2% (w/w), such as in the range of 0.2-1.5% (w/w). Alternatively, the liquid milk product may have a total content of lactose in the range of 0.3-1.0% (w/w), such as approx. 0.4% (w/w).

The lactose concentration (% relative to total DP2) may e.g. be determined by high performance anion exchange chromatography coupled with pulsed amperometric detector (HPAEC-PAD). This method can quantify lactose concetration in milk (mg/L) and can also quantify the % of lactose relative to total disaccharides (DP2). The quantification of % lactose, relative to total DP2, is important in the quantification of lactose in GOS mixtures, as lactose co-elutes with other disaccharides, including the transgalactosylated disaccharides, in the same peak using traditional HPLC methods. For example, a Dionex system consisting of a GS50 gradient pump, an ED50 electrochemical detector with a gold working electrode, an LC25 chromatography oven, and an AS50 autosampler may be used. Separation may be performed using a pellicular anion-exchange resin based column, CarboPac PA-1 analytical (4mm×250 mm), connected to a CarboPac PA1 Guard (4mm×50mm). The column is maintained at 25 °C; the elution is performed at a flow rate of 1ml/min using gradient elution.

The following 4 eluents are preferably used: A: 8 mM NaOH; B: 125 mM NaOH; C: 125 mM NaOH and 400 mM (sodium acetate); D: 8 mM sodium acetate. These four eluents are mixed in a gradient mode so that traces of NaOH are present in the first few minutes of elution. After 4-5 minutes, the concentration of NaOH and sodium acetate increases gradually to allow the elution of disaccharides, trisaccharides, tetra- and so on. All the sodium hydroxide solutions have a pH > 8 and mostly > 10. Sodium acetate solution alone has a pH of approx. 8.

Under these conditions, lactose is eluted as a separate peak from the other disaccharides, which allowed its quantification using a standard calibration curve and appropriate DP2 standards. All chromatographic analyses should be performed in duplicates.

The GOS-DP2 fraction to be analysed may be isolated from the total carbohydrate population by preparative gel filtration methods.

In some embodiments of the invention the liquid milk product has a total content of galactose and glucose of at most 10% (w/w). The liquid milk product may have a total content of galactose and glucose of at most 10% (w/w). The liquid milk product may e.g. have a total content of galactose and glucose of at most 5% (w/w), such as at most 3% (w/w). Alternatively, the liquid milk product may have a total content of galactose and glucose of at most 2% (w/w), such as at most 1% (w/w).

The liquid milk product may e.g. have a total content of galactose and glucose in the range of 0.1-10% (w/w). For example, the liquid milk product may have a total content of galactose and glucose in the range of 0.3-8% (w/w), such as in the range of 0.5-6% (w/w). Alternatively, the liquid milk product may have a total content of galactose and glucose in the range of 1-3% (w/w), such as approx. 2% (w/w).

Normally, the liquid milk product contains at least some glucose and galactose. For example, the liquid milk product typically contains at least 0.1% (w/w) glucose and at least 0.1% (w/w) galactose.

In some preferred embodiments of the invention the liquid milk product contains a total amount of glucose in the range of 0.5-3% (w/w) and a total amount of galactose in the range of 0.1-1.0%. For example, the liquid milk product may contain a total amount of glucose in the range of 0.8-2.0% (w/w) and a total amount of galactose in the range of 0.2-0.6% (w/w).

In some preferred embodiments of the invention the liquid milk product has a total content of monosaccharide of at most 3.0% (w/w). The liquid milk product may have a total content of monosaccharide of at most 2.5% (w/w). The liquid milk product may e.g. have a total content of monosaccharide of at most 2.0% (w/w), such as at most 1.5% (w/w). Alternatively, the liquid milk product may have a total content of monosaccharide of at most 1.0% (w/w), such as at most 0.5% (w/w).

The liquid milk product may e.g. have a total content of monosaccharide in the range of 0.1-3.0% (w/w). For example, the liquid milk product may have a total content of monosaccharide in the range of 0.2-2.5% (w/w), such as in the range of 0.5-2.0% (w/w). Alternatively, the liquid milk product may have a total content of monosaccharide in the range of 0.1-2.0% (w/w), such as approx. 1.5% (w/w).

The present inventors have found that liquid milk products of the present invention having a relatively low content of monosaccharide are particularly advantageous for uses which involve high temperature heating or even conversion of the liquid milk product into powder and provide products having a better long-term stability than GOS-containing milk products having a higher content of monosaccharides. Without being bound by theory, it is believed that a higher level of monosaccharides gives rise to more Maillard reaction which is an undesirable chain reaction initiated by reducing saccharides and proteins containing primary amino groups (e.g. through lysine residues). The Maillard reaction may reduce the nutritional value of the milk product and may spoil its colour and taste.

In some embodiments of the invention the liquid milk product has a total content of galactose of at most 3% (w/w). The liquid milk product may have a total content of galactose of at most 2.5% (w/w). The liquid milk product may e.g. have a total content of galactose of at most 2.0% (w/w). Alternatively, the liquid milk product may have a total content of galactose of at most 1.5% (w/w).

In some preferred embodiments of the invention the liquid milk product has a total content of galactose of at most 1.0% (w/w). The liquid milk product may have a total content of galactose of at most 0.5% (w/w). The liquid milk product may e.g. have a total content of galactose of at most 0.4% (w/w), such as at most 0.3% (w/w). Alternatively, the liquid milk product may have a total content of galactose of at most 0.2% (w/w), such as at most 0.1% (w/w).

The liquid milk product may e.g. have a total content of galactose in the range of 0.05-3% (w/w). For example, the liquid milk product may have a total content of galactose in the range of 0.1-2% (w/w), such as in the range of 0.2-1.5% (w/w). Alternatively, the liquid milk product may have a total content of galactose in the range of 0.3-1.0% (w/w), such as approx. 0.4% (w/w).

The liquid milk product preferably has a total content of GOS of at least 0.9% (w/w), and preferably at least 1.0% (w/w). The liquid milk product may have a total content of GOS of at least 1.2% (w/w). The liquid milk product may e.g. have a total content of GOS of at least 1.4% (w/w). Alternatively, the liquid milk product may have a total content of GOS of at least 2.0% (w/w).

In some preferred embodiments of the invention the liquid milk product has a total content of GOS of at least 2.2% (w/w). The liquid milk product may have a total content of GOS of at least 2.4% (w/w). The liquid milk product may e.g. have a total content of GOS of at least 2.6% (w/w), such as at least 2.8% (w/w). Alternatively, the liquid milk product may have a total content of GOS of at least 3.0% (w/w), such as at least 3.5% (w/w).

The liquid milk product may e.g. have a total content of GOS in the range of 0.9-10% (w/w). Alternatively, the liquid milk product may have a total content of GOS in the range of 1-10% (w/w). For example, the liquid milk product may have a total content of GOS in the range of 1.0-8.0% (w/w), such as in the range of 1.2-6.0% (w/w). Alternatively, the liquid milk product may have a total content of GOS in the range of 1.4-5.0% (w/w), such as approx. 2% (w/w).

It is preferred that the liquid milk product comprises a significant amount of GOS having a degree of polymerisation (DP) of 3 and higher. A GOS molecule having a DP of 3 (DP3) is a tri-saccharide, a GOS molecule having a DP of 4 (DP4) is a tetra-saccharide and so forth.

It is thus preferred that the liquid milk product comprises a total amount of GOS-DP3+ of at least 0.5% (w/w). In the context of the present invention, the term "GOS-DPx+" means the sum of GOS molecules having DPx or higher. For example, the term "DP3+" means the sum of GOS molecules having DP3, DP4, DP5, DP6, etc.

The liquid milk product preferably has a total content of GOS-DP3+ of at least 0.9% (w/w), and even more preferably at least 1.0% (w/w). The liquid milk product may have a total content of GOS-DP3+ of at least 1.2% (w/w). The liquid milk product may e.g. have a total content of GOS-DP3+ of at least 1.4% (w/w). Alternatively, the liquid milk product may have a total content of GOS-DP3+ of at least 2.0% (w/w).

A relatively high content of GOS and particularly GOS-DP3+ is believed to increase the prebiotic effect of the liquid milk product relative to liquid milk products of the prior art.

In some preferred embodiments of the invention the liquid milk product has a total content of GOS-DP3+ of at least 2.2% (w/w). The liquid milk product may have a total content of GOS-DP3+ of at least 2.4% (w/w). The liquid milk product may e.g. have a total content of GOS-DP3+ of at least 2.6% (w/w), such as at least 2.8% (w/w). Alternatively, the liquid milk product may have a total content of GOS-DP3+ of at least 3.0% (w/w), such as at least 3.5% (w/w).

The liquid milk product may e.g. have a total content of GOS-DP3+ in the range of 0.5-10% (w/w). For example, the liquid milk product may have a total content of GOS-DP3+ in the range of 1.0-8.0% (w/w), such as in the range of 1.2-6.0% (w/w). Alternatively, the liquid milk product may have a total content of GOS-DP3+ in the range of 1.4-5.0% (w/w), such as approx. 2% (w/w).

In the context of the present invention, the term "GOS-DPx" means the sum of GOS molecules having DPx. For example, the term "DP3" means the sum of GOS molecules having DP3, but excludes GOS having a higher or lower DP.

In some preferred embodiments of the invention the liquid milk product has a weight ratio between GOS-DP4+ and GOS-DP3 of at least 0.15.

Liquid milk products having a higher ratio between GOS-DP4+ and GOS-DP3 typically contain a broader variety of GOS-DP4 species, GOS-DP5 species, GOS-DP6 species and GOS-DP7 species than liquid milk products having lower ratio between GOS-DP4+ and GOS-DP3 and are therefore believed to provide a better, more diverse prebiotic effect and are believed to be fermented at a slower rate than GOS-DP2 and GOS-DP3 alone. The high diversity of degrees of polymerisation and the high diversity of the species within each DP cluster, is believed to provide a wider efficacy on more probiotic bacterial strains and species within the healthy microbiota species of the gastrointestinal tract.

In other preferred embodiments of the invention the liquid milk product has a weight ratio between GOS-DP4+ and GOS-DP3 of at least 0.2. For example, the weight ratio between GOS-DP4+ and GOS-DP3 may be at least 0.25.

In other preferred embodiments of the invention the liquid milk product has a weight ratio between GOS-DP4+ and GOS-DP3 of at least 0.30. Even more preferred, the weight ratio between GOS-DP4+ and GOS-DP3 may be at least 0.4.

In some preferred embodiments of the invention the liquid milk product has a weight ratio between GOS-DP4+ and GOS-DP3 in the range of 0.15-2.

In other preferred embodiments of the invention the liquid milk product has a weight ratio between GOS-DP4+ and GOS-DP3 in the range of 0.2-1. Even more preferably the weight ratio between GOS-DP4+ and GOS-DP3 may be in the range of 0.25-1.0.

In other preferred embodiments of the invention the liquid milk product has a weight ratio between GOS-DP4+ and GOS-DP3 may be in the range of 0.30-0.8. Even more preferred the weight ratio between GOS-DP4+ and GOS-DP3 may be in the range of 0.4-0.6.

In some preferred embodiments of the invention the liquid milk product has a weight ratio between GOS-DP5 and GOS-DP3 of at least 0.01.

In other preferred embodiments of the invention the liquid milk product has a weight ratio between GOS-DP5 and GOS-DP3 of at least 0.02. For example, the weight ratio between GOS-DP5 and GOS-DP3 may be at least 0.03.

In other preferred embodiments of the invention the liquid milk product has a weight ratio between GOS-DP5 and GOS-DP3 of at least 0.04. Even more preferred, the weight ratio between GOS-DP5 and GOS-DP3 may be at least 0.5.

In some preferred embodiments of the invention the liquid milk product has a weight ratio between GOS-DP5 and GOS-DP3 in the range of 0.01-0.2.

In other preferred embodiments of the invention the liquid milk product has a weight ratio between GOS-DP5 and GOS-DP3 in the range of 0.02-0.15. Even more preferably the weight ratio between GOS-DP5 and GOS-DP3 may be in the range of 0.03-0.1.

In other preferred embodiments of the invention the liquid milk product has a weight ratio between GOS-DP5 and GOS-DP3 may be in the range of 0.03-0.09. The weight ratio between GOS-DP5 and GOS-DP3 may e.g. be in the range of 0.04-0.08.

In some preferred embodiments of the invention the liquid milk product has a weight ratio between glucose and galactose of at least 3:1, and preferably at least 3.0:1.0. For example, the weight ratio between glucose and galactose may be at least 3.5:1.0. The weight ratio between glucose and galactose may e.g. be at least 4.0:1.0, such as at least 4.5:1.0. Alternatively, the weight ratio between glucose and galactose may be at least 5.0:1.0, such as at least 6.0:1.0.

Protein is an important nutritional component in the liquid milk product, and in an embodiment of the invention the liquid milk product comprises a total amount of protein in the range of 1-10% (w/w). For example, the liquid milk product may comprise a total amount of protein in the range of 2-8% (w/w). For example, the liquid milk product may comprise a total amount of protein in the range of 2.5-6% (w/w). Alternatively, the liquid milk product may comprise a total amount of protein in the range of 3.0-5% (w/w).

The total amount of protein is preferably determined according to ISO 8968-3:2004 (Determination of nitrogen content Block-digestion method).

Casein is normally the predominant protein in milk products, and in an embodiment of the invention the liquid milk product comprises a total amount of casein in the range of 0.5-8% (w/w). For example, the liquid milk product may comprise a total amount of casein in the range of 1-6% (w/w), and preferably in the range of 2.0-5% (w/w), and even more preferably in the range of 2.5-4% (w/w).

Casein is normally the predominant protein in milk, and in some embodiments of the invention the liquid milk product comprises a total amount of casein in the range of 0.5-8% (w/w). For example, the liquid milk product may comprise a total amount of casein in the range of 1-6% (w/w), and preferably in the range of 2.0-5% (w/w), and even more preferably in the range of 2.5-4% (w/w).

In addition to casein, the liquid milk product typically contains native milk serum proteins, such as beta-lactoglobulin, alpha-lactalbumin, serum albumin, immunoglobins, etc. In some preferred embodiments of the invention the liquid milk product comprises a total amount of milk serum protein in the range of 0.2-4% (w/w). For example, the liquid milk product may comprise a total amount of milk serum protein in the range of 0.4-3% (w/w), and preferably in the range of 0.5-2% (w/w), and even more preferably in the range of 0.6-1.5% (w/w).

In some preferred embodiments of the invention the liquid milk product has a furosine value of at most 60 mg/100 g protein 7 days after the production when kept at a temperature of 25 degrees C during storage. Preferably, the liquid milk product has a furosine value of at most 55 mg/100 g protein 7 days after the production when kept at a temperature of 25 degrees C during storage. Even more preferably, the liquid milk product may have a furosine value of at most 50 mg/100 g protein 7 days after the production when kept at a temperature of 25 degrees C during storage.

In other preferred embodiments of the invention the liquid milk product has a furosine value of at most 40 mg/100 g protein 7 days after the production when kept at a temperature of 25 degrees C during storage. Preferably, the liquid milk product has a furosine value of at most 30 mg/100 g protein 7 days after the production when kept at a temperature of 25 degrees C during storage. Even more preferably, the liquid milk product may have a furosine value of at most 20 mg/100 g protein 7 days after the production when kept at a temperature of 25 degrees C during storage.

In some preferred embodiments of the invention the liquid milk product comprises at least 0.20 mmol available $NH_2$-groups per g milk serum protein.

The amount of available $NH_2$-groups per g milk serum protein may be determined as outlined to F. Chevalier et al. (International Dairy Journal 11 (2001) 145-152). The milk serum phase may be isolated from the milk product by acid precipitation of casein and subsequent dialysis of the milk serum phase against 0.1 mol/L phosphate buffer, pH 6.5.

For example, the liquid milk product may comprise at least 0.25 mmol available $NH_2$-groups per g milk serum protein. Preferably, the liquid milk product comprises at least 0.25 mmol available $NH_2$-groups per g milk serum protein. The liquid milk product may e.g. comprise at least 0.30 mmol available $NH_2$-groups per g milk serum protein.

In other preferred embodiments of the invention the liquid milk product comprises at least 0.35 mmol available $NH_2$-groups per g milk serum protein. For example, the liquid milk product may comprise at least 0.40 mmol available $NH_2$-groups per g milk serum protein. Preferably, the liquid milk product comprises at least 0.45 mmol available $NH_2$-groups per g milk serum protein. The liquid milk product may e.g. comprise at least 0.50 mmol available $NH_2$-groups per g milk serum protein.

Fats are yet an important nutritional component in the liquid milk product and may additionally be the carrier of fat-soluble vitamins. In an embodiment of the invention, the liquid milk product comprises a total amount of fat in the range of 0.01-40% (w/w). For example, the liquid milk product may comprise a total amount of fat in the range of 0.1-20% (w/w). Alternatively, the liquid milk product may comprise a total amount of fat in the range of 0.5-5% (w/w). For example, the liquid milk product may comprise a total amount of fat in the range of 1-4% (w/w).

The present invention also allows for low fat liquid milk products. Thus, in an embodiment of the invention, the liquid milk product comprises a total amount of fat in the range of 0.01-1.0% (w/w). For example, the liquid milk product may comprise a total amount of fat in the range of 0.05-0.6% (w/w). Alternatively, the liquid milk product may comprise a total amount of fat in the range of 0.1-0.4% (w/w).

The fat of the liquid milk product preferably comprises, or even consists of, milk fat. However, in some embodiment of the invention, the fat also comprises vegetable fat. Non-limiting examples of useful vegetable fat include maize oil, sesame oil, soybean oil, linseed oil, grapeseed oil, rapeseed oil, olive oil, groundnut oil, sunflower oil, safflower oil, palm oil, palm kernel oil, coconut oil, and a combination thereof.

In the context of the present invention the terms fat and oil are used interchangeably.

The solid fat content may be determined according to ISO 8292-1&2:2008 or ISO 1736:2008 (Dried milk and milk products - Determination of fat content - Gravimetric method (Reference method)).

The liquid milk product may furthermore contain the minerals that are typically present in ruminant milk and it may furthermore have been supplemented with additional minerals.

In some embodiments of the invention the sodium content of the liquid milk product is at most 300 mg/100 g liquid milk product, preferably at most 200 mg/100 g liquid milk product, and even more preferably at most 100 mg/100 g liquid milk product, such as e.g. in the range of 30-60 mg/100 g liquid milk product.

In some preferred embodiments of the invention, the solids content of the liquid milk product is preferably at most 25% (w/w) relative to the total weight of the product. For example, the liquid milk product may have a solids content of at most 20% (w/w). The solids content of the liquid milk product may e.g. be at most 15% (w/w). Alternatively, the solids content of the liquid milk product may e.g. be at most 10% (w/w).

For example, the liquid milk product may have a solids content of at most 20% (w/w). The solids content of the liquid milk product may e.g. be at most 15% (w/w). Alternatively, the solids content of the liquid milk product may e.g. be at most 10% (w/w).

In some preferred embodiments of the invention, the liquid milk product has a solids content in the range of 2-30% (w/w) relative to the total weight of the liquid milk product. Preferably, the liquid milk product may have a solids content in the range of 4-20% (w/w). Even more preferably, the liquid milk product may have a solids content in the range of 6-16% (w/w), such as e.g. in the range of 7-14% (w/w) relative to the total weight of the milk product.

The pH of the liquid milk product may be weak acidic, neutral or basic. The pH is preferably at least 5, and preferably at least 6, such as approx. 7. In some preferred embodiments of the invention the pH of the liquid milk product is in the range of 5.0-9. For example, the pH of the liquid milk product may be in the range 6.0-8. The pH of the liquid milk product may e.g. be in the range 6.5-7.5. Unless mentioned otherwise, all pH values described herein are measured at 10 degrees C.

In some embodiments of the invention, the liquid milk product is a liquid milk product which is not enriched by addition of pre-produced galacto-oligosaccharides.

In a preferred embodiment of the invention the liquid milk product has a pH in the range of 5.0-8, the liquid milk product comprising:

- at least 1.0% (w/w) GOS,
- at most 1% (w/w) lactose,
- a total content of monosaccharide of at most 3.0% (w/w), and
- a total amount of carbohydrate in the range 3-6% (w/w).

In other preferred embodiments of the invention, the liquid milk product, e.g. obtainable by the method defined herein, has a pH in the range of 5.0-8, and comprises:

- at least 1.0% (w/w) GOS,
- at most 1% (w/w) lactose,
- a total content of monosaccharide of at most 3.0% (w/w),
- a total amount of carbohydrate in the range 3-6% (w/w), and
- a weight ratio between glucose and galactose of at least 2:1, and preferably at least 3:1.

In yet other preferred embodiments of the invention, the liquid milk product, e.g. obtainable by the method defined herein, has a pH in the range of 5.0-8, and comprises:

- at least 1.0% (w/w) GOS,
- at most 1% (w/w) lactose,
- a total content of monosaccharide of at most 3.0% (w/w),
- a total amount of carbohydrate in the range 3-6% (w/w), and
- a contain a total amount of glucose in the range of 0.8-2.0% (w/w) and a total amount of galactose in the range of 0.2-0.6% (w/w).

In a preferred embodiment of the invention the liquid milk product, e.g. obtainable by the method defined herein, has a pH in the range of 5.0-8, the liquid milk product comprising:

- at least 1.0% (w/w) GOS,
- a total content of GOS-DP3+ of at least 0.9% (w/w)
- at most 1% (w/w) lactose,
- a total content of monosaccharide of at most 3.0% (w/w), and
- a total amount of carbohydrate in the range 3-6% (w/w).

In other preferred embodiments of the invention, the liquid milk product, e.g. obtainable by the method defined herein, has a pH in the range of 5.0-8, and comprises:

- at least 1.0% (w/w) GOS,
- a total content of GOS-DP3+ of at least 0.9% (w/w),
- at most 1% (w/w) lactose,
- a total content of monosaccharide of at most 3.0% (w/w),
- a total amount of carbohydrate in the range 3-6% (w/w), and
- a weight ratio between glucose and galactose of at least 2:1, and preferably at least 3:1.

In yet other preferred embodiments of the invention, the liquid milk product, e.g. obtainable by the method defined herein, has a pH in the range of 5.0-8, and comprises:

- at least 1.0% (w/w) GOS,
- a total content of GOS-DP3+ of at least 0.9% (w/w)
- at most 1% (w/w) lactose,
- a total content of monosaccharide of at most 3.0% (w/w),
- a total amount of carbohydrate in the range 3-6% (w/w), and
- a total amount of glucose in the range of 0.8-2.0% (w/w) and a total amount of galactose in the range of 0.2-0.6% (w/w).

The fat and protein composition of such a liquid milk product is preferably similar to that of normal low fat milk, such as e.g. skimmed milk, the main difference being that the present liquid milk product may contain inactivated enzyme.
The visual appearance of the liquid milk product may e.g. be similar to that of low fat milk or skim milk or that of a flavoured low fat milk or skim milk.
In some preferred embodiments of the invention, the liquid milk product is sterile, e.g. obtained by UHT treatment or a similar treatment which kills all microorganisms. In the context of the present invention the term "sterile" means that the product in question does not contain any viable microorganisms.
In some preferred embodiments of the invention, the liquid milk product has a shelf-life of at least 180 days after production when stored at 25 degrees C, preferably at least 240 day after production when stored at 25 degrees C, and even more preferred at least 360 days after production when stored at 25 degrees C. Such a long shelf-life liquid milk may for example have been subjected to UHT-treatment or a similar heat-treatment and to plasmin inactivation.
Yet an aspect of the invention pertains to a powdered milk product containing the solids of the liquid milk product. For example, powdered milk product may consist essentially of the solids of the liquid milk product and water.
In some preferred embodiments of the invention the powdered milk product contains solids of the liquid milk product in an amount of 90-97% (w/w) and water in an amount of 3-10% (w/w).
Such a powdered milk product may be obtained by spray-drying the liquid milk product.
In some preferred embodiments of the invention the powdered milk product contains the solids of liquid milk product as described herein having a solids content of 12% (w/w). This means that an embodiment relating to the concentration of solids components of the liquid milk product can be converted to the composition of the solids of the powdered milk product by multiplying the concentration of solids component mentioned in the context of the liquid milk product with a factor of 8.3 (=100%/12%).
Thus, if the liquid milk product having a solids content of 12% (w/w) comprises

- at least 1.0% (w/w) GOS,
- at most 3% (w/w) lactose,
- at total amount of glucose and galactose of at least 0.1% (w/w), and
- a weight ratio between glucose and galactose of at least 3:1,

the solids of the corresponding powdered milk product would comprise

- at least 8.3% (w/w) GOS relative to the total weight of the solids,
- at most 25% (w/w) lactose relative to the total weight of the solids,
- at total amount of glucose and galactose of at least 0.8% (w/w) relative to the total weight of the solids, and
- a weight ratio between glucose and galactose of at least 3:1.

The pH which is obtained when the 10 g of powdered milk product is dissolved in 90 g demineralised water is at least 5.0 and may be the same as described in the context of the liquid milk product.
The liquid or powdered milk product is preferably present in a sealed container.

The present inventors have discovered that the above liquid milk products can be produced by low temperature, enzymatic GOS-synthesis directly in the milk with a surprisingly high yield of GOS.

Thus, another aspect of the invention pertains to a method of producing a galacto-oligosaccharide-containing milk product, the method comprising the steps of:

a) providing an enzyme having trans-galactosylation activity,

b) providing a milk-derived composition containing lactose in an amount of at most 10% (w/w), which milk-derived composition has a pH of at least 5 relative to the total weight of the solids,

c) contacting the milk-derived composition with the enzyme at a temperature of at most 10 degrees C for a duration sufficient for the synthesis of galacto-oligosaccharide (GOS) to take place,

d) inactivating and/or removing at least some of the enzyme,

e) optionally, converting the GOS-containing milk-derived composition obtained from step d) to a powder,

f) optionally, packaging the GOS-containing milk-derived composition, e.g. in liquid form or in powder form.

The present inventors have found that surprisingly it is possible to produce a high yield of GOS in milk using low temperature during the enzymatic treatment.

The method may be implemented in several variants.

In some embodiments of the invention, the method comprises steps a), b), c), and d).

In further embodiments of the invention, the method comprises steps a), b), c), d) and e).

In still further embodiments of the invention, the method comprises steps a), b), c), d), e), and f).

In further embodiments of the invention, the method comprises steps a), b), c), d) and f) but not step e).

In still further embodiments of the invention, the method comprises steps a), b), c), d), and f) but not step e).

Steps are preferably performed in alphabetic order. However, steps a) and b) may be performed both in the order a), b) and in the order b), a).

The enzyme used in the present invention has trans-galactosylation activity, meaning that it is able to transfer the galactosyl group of a donor substrate, such as lactose, to an acceptor molecule which is not water. Preferably, the enzyme has activity on galactosyl linkages which have the beta(1-4) conformation. This effectively puts the enzymes into the IUBMB EC 3.2.1.23 class of beta-galactosidases.

In some preferred embodiments of the invention, the enzyme has a ratio between its trans-galactosylation activity and its hydrolase activity of at least 1, meaning that for each lactose molecule which is split into galactose and glucose, approx. 1 lactose molecule is split into glucose and a galactosyl group which is transferred to a non-water acceptor.

The ratio between the trans-galactosylation activity and the hydrolase activity of an enzyme is preferably determined according to Method 4 of WO 2013/182686.

For example, the enzyme may have a ratio between its trans-galactosylation activity and its hydrolase activity of at least 2. The enzyme may e.g. have a ratio between its trans-galactosylation activity and its hydrolase activity of at least 3. Alternatively, the enzyme may have a ratio between its trans-galactosylation activity and its hydrolase activity of at least 4.

Even higher ratios may be preferred, thus, the enzyme may have a ratio between its trans-galactosylation activity and its hydrolase activity of at least 5. The enzyme may e.g. have a ratio between its trans-galactosylation activity and its hydrolase activity of at least 7. Alternatively, the enzyme may have a ratio between its trans-galactosylation activity and its hydrolase activity of at least 10.

As mentioned above, the first enzyme has transgalactosylating activity in addition to beta-galactosidase activity. It may also be preferred that the first enzyme has a T-value of at most 0.9.

In the context of the present invention the term "transgalactosylating activity" of a beta-galactosidase enzyme relates to the ability of the enzyme to transfer a galactosyl group from a donor molecule, e.g. a lactose molecule, to a non-water molecule, e.g. another lactose molecule.

The T-value is an alternative measure of the transgalactosylating efficiency of a beta-galactosidase enzyme using lactose as both galactosyl donor and acceptor. The determination of the T-value of a beta-galactosidase enzyme is performed according to the assay and the formula described in Example 4. The T-value is calculated using the formula:

$$\text{T-value} = \text{amount of produced galactose : amount of used lactose}$$

Both the amount of produced galactose and the amount of used lactose should be in the unit mol in order to determine the T-value.

A lactase enzyme without any transgalactosylating activity will produce one mol galactose for each used mol lactose and would have a T-value of 1. A beta-galactosidase having an extremely high transgalactosylating activity would use nearly all the galactosyl groups from the lactose for transgalactosylating instead of generating galactose and would consequently have a T-value near 0.

It is preferred that the enzyme has a T-value of at most 0.6. In some embodiments of the invention, the T-value of the enzyme is at most 0.5, preferably at most 0.4, and even more preferably at most 0.3. For example, the T-value of the enzyme may be at most 0.2. Preferably, the T-value of the enzyme may be at most 0.1. It may even be more preferred that the T-value of the enzyme is at most 0.05.

Non-limiting examples of useful enzymes may be found in the patent applications WO 2001/90317 A2, WO 2013/182686 A1, WO 2011/120993 A1 which are all incorporated herein by reference for all purposes.

In some preferred embodiments of the invention the enzyme is selected from the group consisting of:

- a polypeptide having a sequence identity of at least 80% relative to the polypeptide having the sequence from position 1 (Val) to 1142 (Ile) of SEQ ID NO 1,
- a polypeptide having a sequence identity of at least 80% relative to the polypeptide having the sequence from position 1 (Val) to 887 (Thr) of SEQ ID NO 1, and
- a polypeptide having a sequence identity of at least 80% relative to the polypeptide having the sequence from position 1 (Val) to 965 (Leu) of SEQ ID NO 1.

Truncates of the mature extracellular lactase (SEQ ID NO 1 - corresponding to the sequence from position 33 (Val) to position 1752 (Gly) of UniProt-sequence UPI00001B62C5, www.uniprot.org/) from bifidum DSM20215 have been found to have high levels of trans-galactosylation activity.

In the context of the present invention the term "sequence identity" relates to a quantitative measure of the degree of identity between two amino acid sequences of equal length or between two nucleic acid sequences of equal length. If the two sequences to be compared are not of equal length, they must be aligned to the best possible fit. The sequence identity can be calculated as

$$(Nref-Ndif)*100)/(Nref),$$

wherein Ndif is the total number of non-identical residues in the two sequences when aligned, and wherein Nref is the number of residues in one of the sequences. Hence, the DNA sequence AGTCAGTC will have a sequence identity of 75% with the sequence AATCAATC (Ndif=2 and Nref=8). A gap is counted as non-identity of the specific residue(s), i.e. the DNA sequence AGTGTC will have a sequence identity of 75% with the DNA sequence AGTCAGTC (Ndif=2 and Nref=8). Sequence identity can for example be calculated using appropriate BLAST-programs, such as the BLASTp-algorithm provided by National Center for Biotechnology Information (NCBI), USA.

In other preferred embodiments of the invention the amino acid sequence of the enzyme has a sequence identity of at least 80% relative to the peptide of SEQ ID NO. 1. For example, the amino acid sequence of the first enzyme may have a sequence identity of at least 90% relative to the peptide of SEQ ID NO. 2, preferably at least 95%, and even more preferably at least 97.5%. In some instances the amino acid sequence of the first enzyme may have a sequence identity of at least 99% relative to the peptide of SEQ ID NO. 2.

In some preferred embodiments of the invention the enzyme is a polypeptide having a sequence identity of at least 90% relative to the polypeptide having the sequence from position 1 (Val) to 887 (Thr) of SEQ ID NO 1. Preferably, the enzyme is a polypeptide having at least a sequence identity of at least 95% relative to the polypeptide having the sequence from position 1 (Val) to 887 (Thr) of SEQ ID NO 1. Even more preferred, the enzyme is a polypeptide having a sequence identity of at least 98% relative to the polypeptide having the sequence from position 1 (Val) to 887 (Thr) of SEQ ID NO 1.

In some preferred embodiments of the invention the enzyme is a polypeptide having the sequence from position 1 (Val) to 887 (Thr).

In some preferred embodiments of the invention the enzyme is a polypeptide having a sequence identity of at least 90% relative to the polypeptide having the sequence from position 1 (Val) to 1142 (Ile) of SEQ ID NO 1. Preferably, the enzyme is a polypeptide having at least a sequence identity of at least 95% relative to the polypeptide having the sequence from position 1 (Val) to 1142 (Ile) of SEQ ID NO 1. Even more preferred, the enzyme is a polypeptide having a sequence identity of at least 98% relative to the polypeptide having the sequence from position 1 (Val) to 1142 (Ile) of SEQ ID NO 1.

In some preferred embodiments of the invention the enzyme is a polypeptide having the sequence from position 1 (Val) to 1142 (Ile).

In some preferred embodiments of the invention the enzyme is a polypeptide having a sequence identity of at least 90% relative to the polypeptide having the sequence from position 1 (Val) to 965 (Leu) of SEQ ID NO 1. Preferably, the enzyme is a polypeptide having at least a sequence identity of at least 95% relative to the polypeptide having the sequence from position 1 (Val) to 965 (Leu) of SEQ ID NO 1. Even more preferred, the enzyme is a polypeptide having a sequence identity of at least 98% relative to the polypeptide having the sequence from position 1 (Val) to 965 (Leu) of SEQ ID NO 1.

In some preferred embodiments of the invention the enzyme is a polypeptide having the sequence from position 1 (Val) to 965 (Leu) of SEQ ID NO 1.

The milk-derived composition provided in step b) is preferably a liquid composition.

In the context of the present invention, the term "milk-derived composition" means a composition which contains casein and/or milk serum protein and which furthermore contains lactose and minerals.

In the context of the present invention, the phrase "Y and/or X" means "Y" or "X" or "Y and X". Along the same line of logic, the phrase "$n_1$, $n_2$, ..., $n_{i-1}$, and/or $n_i$" means "$n_1$" or "$n_2$" or ... or "$n_{i-1}$" or "$n_i$" or any combination of the components : $n_1$, $n_2$,...$n_{i-1}$, and $n_i$.

In some preferred embodiments of the invention the milk-derived composition comprises a total amount of non-fat milk solids of at least 25% (w/w) on a solids basis, preferably at least 50% (w/w), even more preferably at least 75% (w/w), such as at least 90% (w/w) on a solids basis.

In some embodiments of the invention the milk-derived composition comprises a total amount of non-fat milk solids of 100% (w/w) on a solids basis.

In some preferred embodiments of the invention, the milk-derived composition has a solids content in the range of 2-30% (w/w) relative to the total weight of the composition, preferably in the range of 4-20% (w/w), and even more preferably in the range of 6-16% (w/w), such as e.g. in the range of 7-14% (w/w) relative to the total weight of the composition.

The milk-derived composition typically comprises a total amount of lactose in the range of 5-80% (w/w) relative to the total amount of non-fat milk solids.

The milk-derived composition may e.g. comprise a total amount of lactose of at most in 10% (w/w) relative to the total weight of the composition. Preferably, the milk-derived composition comprises a total amount of lactose of at most 7% (w/w). For example, the milk-derived composition may comprise a total amount of lactose of at most 5% (w/w) relative to the total weight of the composition, such as at most 3% (w/w).

Except for the enzyme and carbohydrates affected by the GOS synthesis, the milk-derived composition may contain the same components in the same concentrations as the liquid milk product. Thus, the various components and their concentration ranges which are described in the context of the liquid milk products may be present in the milk-derived composition in the same concentration ranges.

The milk-derived composition may for example be a composition selected from the group consisting of whole milk, low fat milk, skim milk, buttermilk, reconstituted milk powder, condensed milk, UHT milk, whey, whey permeate, and cream.

In some preferred embodiments of the invention, the milk-derived composition is a composition selected from the group consisting of whole milk, low fat milk, skim milk, buttermilk, reconstituted milk powder, condensed milk, UHT milk, and cream.

In another embodiment of the invention, the milk-derived composition of step a) comprises at most 40% w/w milk fat. An example of such a milk-derived composition is whipping cream.

In yet an embodiment of the invention, the milk-derived composition of step a) comprises at most 20% w/w milk fat. An example of such a milk-derived composition is single cream/table cream containing approx. 18% w/w milk fat.

In a further embodiment of the invention, the milk-derived composition of step a) comprises at most 4% w/w milk fat. An example of such a milk-derived composition is full fat milk which typically contains 2-4% w/w milk fat, and preferably approx. 3% w/w milk fat.

In a further embodiment of the invention, the milk-derived composition of step a) comprises at most 2% w/w milk fat. An example of such a milk-derived composition is semi-skim milk which typically contains 0.7-2% w/w milk fat, and preferably 1-1.5% w/w milk fat.

In an additional embodiment of the invention, the milk-derived composition of step a) comprises at most 0.7 w/w milk fat. An example of such a milk-derived composition is skim milk which normally contains 0.1-0.7% w/w milk fat, and preferably 0.3-0.6% w/w milk fat, such as approx. 0.5% w/w milk fat

In a preferred embodiment of the invention, the milk-derived composition of step a) comprises at most 0.1% w/w milk fat.

The milk-derived composition may furthermore comprise one or more food ingredient(s). Examples of such food ingredients are a sweetener, a flavouring agent, a pH buffering agent, a stabiliser agent, a colouring agent, an antioxidant, an emulsifier, a mineral, and a vitamin.

The sweetener may comprise carbohydrate sweeteners such as e.g. sucrose, fructose, maltose, and/or mannose, which add to the lactose that is already present in the milk-derived composition.

Alternatively, or additionally, the sweetener may comprise one or more sugar alcohols, such as e.g. sorbitol, mannitol, and/or xylitol.

Alternatively, or additionally, the sweetener may comprise one or more high intensity sweeteners, such as e.g. aspartame, acesulfame, sucralose, saccharin, alitame, and/or neotame.

Step c) involves contacting the milk-derived composition with the enzyme, meaning that the enzyme and milk-derived composition are arranged such that the enzyme is able to convert lactose of the milk-derived composition into GOS.

In some preferred embodiments of the invention the enzyme is simply mixed into the milk-derived composition and will therefore form part, either in active or inactivated form, of the formed GOS-containing milk-derived composition.

In other preferred embodiments of the invention the enzyme is attached to a solids phase which may be brought into contact with the milk-derived composition and subsequently removed from the milk-derived composition again. In such embodiments the GOS-containing milk-derived composition contains no enzyme or only traces of the enzyme.

The temperature of the milk-derived composition during step c) is preferably is in the range of 0-10 degrees C. For example, the temperature of the milk-derived composition during step c) may be in the range of 2-8 degrees C. The temperature of the milk-derived composition during step c) may e.g. be in the range of 3-6 degrees C.

As will be appreciated by the skilled person, few, brief temperature fluctuations which bring the temperature of the milk-derived composition above 10 degrees C may be acceptable as long as they do not provide basis for increased bacterial growth or metabolism.

The duration of the contact of step c) depends on several factors including how much enzyme is used and how much lactose that should be converted to GOS.

The duration of the contact between the enzyme and the milk-derived composition may for example be at most 48 hours. Preferably, the duration of the contact between the enzyme and the milk-derived composition is at most 24 hours. Even more preferably, the duration of the contact between the enzyme and the milk-derived composition is at most 12 hours.

For example, the duration of the contact between the enzyme and the milk-derived composition may for example be in the range of 0.1-48 hours. The duration of the contact between the enzyme and the milk-derived composition may e.g. be in the range of 1-24 hours. Alternatively, the duration of the contact between the enzyme and the milk-derived composition may be in the range of 1-16 hours, such as in the range of 6-14 hours.

When the enzyme is mixed with the milk-derived composition there is an upper limit to how much enzyme the milk-derived composition can contain without affecting the sensory properties of the resulting milk product.

In some preferred embodiments of the invention the enzyme is added to the milk-derived composition in an amount in the range of 1-10000 ppm. For example, the enzyme may be added to the milk-derived composition in an amount in the range of 1-1000 ppm. The enzyme may e.g. be added to the milk-derived composition in an amount in the range of 1-100 ppm.

However, if the enzyme forms part of an enzyme reactor when contacted with the milk-derived composition, a very high enzyme activity may be used, and in such cases the duration of the contact between the enzyme and the milk-derived composition may e.g. be in the range of 0.1-2 hours. For example, the duration of the contact between the enzyme and the milk-derived composition may be in the range of 0.2-1.5 hours. Alternatively, the duration of the contact between the enzyme and the milk-derived composition may be in the range of 0.1-1 hours, such as in the range of 0.2-0.8 hours.

The duration of the contact between the enzyme and the milk-derived composition is preferably sufficient to convert at least 20% of the lactose of the milk-derived composition, reducing the starting amount of lactose in the milk-derived composition to 80% (w/w). For example, if the lactose content of the milk-derived composition provided in step a) was 3.0%, it is thus preferred that at least 20% has been converted and that at most 80%, corresponding to 2.4 (w/w) lactose remains after the enzymatic treatment of step c).

For example, the duration of the contact between the enzyme and the milk-derived composition may be sufficient to convert at least 40% of the lactose of the milk-derived composition. The duration of the contact between the enzyme and the milk-derived composition may e.g. be sufficient to convert at least 60% of the lactose of the milk-derived composition. Alternatively, the duration of the contact between the enzyme and the milk-derived composition may e.g. be sufficient to convert at least 70% of the lactose of the milk-derived composition.

Even higher level lactose conversion may be preferred, thus, the duration of the contact between the enzyme and the milk-derived composition may be sufficient to convert at least 80% of the lactose of the milk-derived composition. For example, the duration of the contact between the enzyme and the milk-derived composition may e.g. be sufficient to convert at least 90% of the lactose of the milk-derived composition. Alternatively, the duration of the contact between the enzyme and the milk-derived composition may e.g. be sufficient to convert at least 95% of the lactose of the milk-derived composition.

In some preferred embodiments of the invention the duration of the contact between the enzyme and the milk-derived composition is sufficient to convert in the range of 20-90% of the lactose of the milk-derived composition. The duration of the contact between the enzyme and the milk-derived composition may e.g. be sufficient to convert in the range of 30-70% of the lactose of the milk-derived composition. Alternatively, the duration of the contact between the enzyme and the milk-derived composition may be sufficient to convert in the range of 40-60% of the lactose of the milk-derived composition.

The present inventors have found that when it is attempted to convert the residual lactose of the GOS-containing milk-derived composition, the content of the monosaccharides increases significantly leading to a poorer stability of the resulting liquid and powdered milk products.

Thus, the duration of the contact between the enzyme and the milk-derived composition is preferably sufficient to convert at most 70% of the lactose of the milk-derived composition.

For example, the duration of the contact between the enzyme and the milk-derived composition may be sufficient to convert at most 60% of the lactose of the milk-derived composition. The duration of the contact between the enzyme and the milk-derived composition may e.g. be sufficient to convert at most 50% of the lactose of the milk-derived composition. Alternatively, the duration of the contact between the enzyme and the milk-derived composition may e.g. be sufficient to convert at most 45% of the lactose of the milk-derived composition. The method preferably involves inactivating and/or removing at least some of the enzyme, thereby reducing the beta-galactosidase (both hydrolase activity and transgalactosylation activity) significantly. Preferably, step d) involves inactivating and/or removing the enzyme completely or at least nearly completely.

The present inventors have found that the mere cooling of the enzyme does not stop its enzymatic activity and that the prolonged storage at low temperature of liquid milk product containing active enzyme will lead to degradation of the synthesized GOS and to an increase in the content of monosaccharides.

The enzyme may for example be inactivated by heat inactivation e.g. by heating the GOS-containing milk-derived composition to a temperature of at least 90 degrees C for at least 10 minutes. For example, the GOS-containing milk-derived composition may be heated to a temperature of at least 95 degrees C for at least 10 minutes.

The heat inactivation may also involve low temperature e.g. starting from 80 degrees C. The beta-galactosidase enzyme may e.g. be inactivated by by heating the GOS-containing milk-derived composition to a temperature of at least 80 degrees C for at least 2 minutes. For example, the GOS-containing milk-derived composition may be heated to a temperature of at least 85 degrees C for at least 5 minutes. Alternatively, the GOS-containing milk-derived composition may be heated to a temperature of at least 80 degrees C for at least 10 minutes.

Alternatively or additionally, step d) may involve heating the GOS-containing milk-derived composition obtained from step c) to at least 140 degrees C for at least 0.1 second, such as e.g. at least 1 second.

The heat treatment step may also involve a high temperature treatment comprising heating the GOS-containing milk-derived composition to a temperature of at least 140 degrees C for at most 5 seconds. For example, a high temperature treatment may comprise heating the GOS-containing milk-derived composition to a temperature in the range of 140-160 degrees C for a duration in the range of 0.5-5 seconds. The high temperature treatment may e.g. comprise heating the GOS-containing milk-derived composition to a temperature in the range of 150-180 degrees C for a duration in the range of 0.1-1 second. Alternatively, a high temperature treatment may comprise heating the GOS-containing milk-derived composition to a temperature in the range of 160-180 degrees C for a duration in the range of 0.1-0.5 seconds.

Alternatively, or additionally, the enzyme may be inactivated by subjecting the GOS-containing milk-derived composition to pressure inactivation.

Preferably, the inactivation provides a reduction of at least 90% of the beta-galactosidase activity of the enzyme. For example, the inactivation may provide a reduction of at least 95% of the beta-galactosidase activity of the enzyme. The inactivation may e.g. provide a reduction of at least 99% of the beta-galactosidase activity of the enzyme. Alternatively, the inactivation may provide a reduction of at least 99.9% of the beta-galactosidase activity of the enzyme.

The GOS-containing milk-derived composition which has been subjected to step d) preferably has a beta-galactosidase activity of at most 1 LAU per kg. For example, it may have a beta-galactosidase activity of at most 0.1 LAU per kg. The GOS-containing milk-derived composition which has been subjected to step d) may e.g. have a beta-galactosidase activity of at most 0.01 LAU per kg. Alternatively, the GOS-containing milk-derived composition which has been subjected to step d) may e.g. have a beta-galactosidase activity of at most 0.001 LAU per kg.

The determination of the beta-galactosidase activity (in the unit of LAU) is preferably performed using Method 3 described in WO 2013/182686. Particulate matter including casein micelles that may interfere with the analysis is preferably removed by filtration.

In addition to the inactivation of the beta-galactosidase enzyme used for GOS synthesis, such temperature treatments also reduce the activity of endogenous milk enzymes, such as plasmin, as well as its pro-enzyme plasminogen.

The enzyme inactivation step should preferably reduce the combined activity of plasmin and plasminogen of the treated liquid by at least 60% relative to the activity of the untreated liquid, preferably by at least 65%, and even more preferably by at least 70%.

The combined activity is a measure of the activity of plasmin in the liquid milk productg plus the activity that can be gained from converting plasminogen into plasmin. The combined activity is determined according to analysis G described in Example I of WO 2012/010699 A1.

Some embodiments of the invention require even lower levels of combined plasmin and plasminogen activity, and for such embodiments the enzyme inactivation step should preferably reduce the combined activity of plasmin and plasminogen of the treated liquid by at least 80% relative to the activity of the untreated liquid, preferably by at least 85% and even more preferably by at least 90%.

In preferred embodiments of the invention, the enzyme inactivation step should reduce the combined activity of plasmin and plasminogen of the treated liquid by at least 95% relative to the activity of the untreated liquid, preferably by at least 97.5%, and even more preferably by at least 99%.

In some embodiments of the invention, the combined activity of plasmin and plasminogen of the liquid milk product is at most 8.000 microUnits/mL, preferably at most 5.000 microUnits/mL and even more preferably at most 3.000 microUnits/mL.

In the context of the present invention, a plasmin activity of one Unit (U) is the plasmin activity which can produce 1 micromol p-Nitroaniline per minute at 25 degrees C, pH 8.9, using Chromozyme PL (Tosyl-Gly-Pro-Lys-4-nitranilide acetate) as substrate.

In other preferred embodiments of the invention, the combined activity of plasmin and plasminogen of the liquid milk product is at most 2.500 microUnits/mL, preferably at most 1.000 microUnits/mL, and even more preferably at most 750 microUnits/mL. It may even be preferred that the combined activity of plasmin and plasminogen of the liquid milk product is at most 600 microUnits/mL, preferably at most 400 microUnits/mL, and even more preferably at most 200 microUnits/mL.

In some preferred embodiments of the invention the GOS-containing milk-derived composition obtained from step d) is converted to a powder.

This conversion may e.g. be performed by common drying technique such as e.g. freeze-drying or spray-drying.

In some embodiments of the invention, step d) forms part of the spray-drying process, meaning that the pre-heating and/or heating which typically is used in spray-drying processes contribute to the heat inactivation of the enzyme.

In other embodiments of the invention, the heat-inactivation includes a separate step d) and subsequent spray-drying in step e), in which case the spray-drying may contribute to additional heat-inactivation of the enzyme.

The spray-drying may be performed using the same parameters that are commonly used for spray-drying for milk products. The temperature of the inlet air may e.g. be in the range of 150-200 degrees C, such as in the range of 160-180 degrees C.

In some preferred embodiments of the invention the GOS-containing milk-derived composition, e.g. in liquid or powder form, is finally packaged.

In this case the GOS-containing milk-derived composition in liquid or powder form makes up the liquid or powdered milk product.

The packaging of step f) may for example be aseptic packaging, i.e. the liquid or powdered milk-related product is packaged under aseptic conditions. For example, the aseptic packaging may be performed by using an aseptic filling system, and it preferably involves filling the milk into one or more aseptic container(s).

The packaging is typically performed at or below room temperature. Thus, the temperature of the liquid or powdered milk product is typically at most 30 degrees C during the packaging, preferably at most 25 degrees C and even more preferably at most 20 degrees C.

The temperature of the liquid or powdered milk product during packaging may for example be in the range of 0-30 degrees C.

It is presently preferred that the temperature of the liquid or powdered milk product during packaging is in the range of 1-10 degrees C.

The liquid milk product may e.g. be packaged in the containers which the consumer will buy. Useful, but non-limiting, examples of such containers are bottles, cans, bags, pouches, or sachets.

Also, the powdered milk product may be packaged in the containers which the consumer will buy. Useful, but non-limiting, examples of such containers are cans, bags, pouches, or sachets.

Yet an aspect of the invention pertains to a method of producing a food product, including both beverages and solid foods, the method comprising the steps of:

i) providing a GOS-containing, liquid milk product and/or a GOS-containing, powdered milk product,

ii) optionally, providing one or more additional ingredients,

iii) converting the GOS-containing milk product and optionally also one or more additional ingredient(s) to the foods product, and

iv) optionally, packaging the food product.

General methods of converting milk products and other ingredients to food products are well-known to the person skilled in the art.

Another aspect of the invention pertains to a food product containing the GOS-containing milk product. For example, the food product may be obtainable by the above method of producing a food product.

In some embodiments of the invention the food product is an acidified dairy product. The acidified dairy product may for example be a yoghurt or a sour cream. If the acidified dairy product is a yoghurt it may be a set yoghurt, a stirred yoghurt or a drinking yoghurt.

In some embodiments of the invention the food product is a cheese. The cheese may for example be a white cheese, a yellow cheese, a fresh cheese (cream cheese), or a process cheese.

In some embodiments of the invention the food product is a beverage. For example, the beverage may be a flavoured milk, a liquid meal replacement, or a whey beverage.

In some embodiments of the invention the food product is a solid food product. For example, the solid food product may be a protein bar, a meal replacement, or milk pasta.

Yet an aspect of the invention pertains to the use of a beta-galactosidase enzyme as defined herein, preferably having:

- a T-value of at most 0.6, and/or
- a ratio between its trans-galactosylation activity and its hydrolase activity of at least 1,

in the production of lactose-reduced milk products to reduce the level of maillard-reaction in the final milk products and/or to extend the shelf-life of the final milk products.

The use may for example relate to production of a milk product which is a UHT-treated, low lactose milk or lactose-free milk. In the context of the present invention the term "low lactose milk" pertains to a milk that contains at most 1 g lactose per 100 g milk and a lactose-free milk pertains to a milk which contains at most 0.1 g lactose per 100 g milk, and preferably at most 0.01 g lactose per 100 g milk.

The invention is further summarized in the following embodiments:

1. A method of producing a galacto-oligosaccharide-containing milk product having a pH of at least 5.0, the method comprising the steps of:

   a) providing an enzyme having trans-galactosylation activity,

   b) providing a milk-derived composition containing lactose in an amount of at most 10% (w/w), which milk-derived composition has a pH of at least 5.0,

   c) contacting the milk-derived composition with the enzyme at a temperature of at most 10 degrees C for a duration sufficient for the synthesis of galacto-oligosaccharide (GOS) to take place,

   d) inactivating and/or removing at least some of the enzyme,

   e) optionally, converting the GOS-containing milk-derived composition to a powder,

   f) optionally, packaging the GOS-containing milk-derived composition, e.g. in liquid form or in powder form.

2. The method according to embodiment 1 wherein the enzyme has a ratio between its trans-galactosylation activity and its hydrolase activity of at least 1.

3. The method according to embodiment 1 or 2 wherein the enzyme is selected from the group consisting of:

   - a polypeptide having a sequence identity of at least 80% relative to the polypeptide having the sequence from position 1 (Val) to 1142 (Ile) of SEQ ID NO 1,
   - a polypeptide having a sequence identity of at least 80% relative to the polypeptide having the sequence from position 1 (Val) to 887 (Thr) of SEQ ID NO 1, and
   - a polypeptide having a sequence identity of at least 80% relative to the polypeptide having the sequence from position 1 (Val) to 965 (Leu) of SEQ ID NO 1.

4. The method according to any of the preceding embodiments, wherein the milk-derived composition comprises a total amount of non-fat milk solids of at least 25% (w/w) on a solids basis.

5. The method according to any of the preceding embodiments, wherein the milk-derived composition has a solids content in the range of 2-30% (w/w) relative to the total weight of the composition.

6. The method according to any of the preceding embodiments, wherein the milk-derived composition is a composition selected from the group consisting of whole milk, low fat milk, skim milk, buttermilk, reconstituted milk powder, condensed milk, UHT milk, whey, whey permeate, and cream.

7. The method according to any of the preceding embodiments, wherein step d) involves heat-inactivation of the enzyme.

8. The method according to any of the preceding embodiments, wherein step d) involves heating the GOS-containing milk-derived composition obtained from step c) to at least 140 degrees C for at least 0.1 second, such as e.g. at least 1 second.

9. The method according to any of the preceding embodiments, containing a step e) of converting the GOS-containing milk-derived composition to a powder.

10. The method according to any of the preceding embodiments, containing a step f) of packaging the GOS-containing milk-derived composition.

11. A liquid milk product having a pH of at least 5.0 comprising

- at least 0.9% (w/w) GOS,
- at most 1.5% (w/w) lactose,
- a total content of monosaccharide of at most 3.0% (w/w), and
- a weight ratio between glucose and galactose of least 2:1.

12. The liquid milk product according to embodiment 11 comprising at least 1.0% (w/w) GOS.

13. The liquid milk product according to embodiment 11 or 12 comprising at most 1.0% (w/w) lactose.

14. The liquid milk product according to any of the embodiments 11-13 having a total content of monosaccharide of at most 2.0% (w/w).

15. The liquid milk product according to any of the embodiments 11-14 having a total amount of glucose in the range of 0.8-2.0% (w/w) and a total amount of galactose in the range of 0.2-0.6% (w/w).

16. The liquid milk product according to any of the embodiments 11-15 having a weight ratio between glucose and galactose of least 3:1.

17. The liquid milk product according to any of the embodiments 11-16 comprising inactivated beta-galactosidase enzyme.

18. The liquid milk product according to any of the embodiments 11-17 comprising a total amount of GOS-DP3+ of at least 0.5% (w/w).

19. The liquid milk product according to any of the embodiments 11-18 wherein the weight ratio between GOS-DP4+ and GOS-DP3 is at least 0.15.

20. The liquid milk product according to any of the embodiments 11-19 wherein the weight ratio between GOS-DP5 and GOS-DP3 is at least 0.01.

21. The liquid milk product according to any of the embodiments 11-20 having a furosine value of at most 60 mg/100 g protein 7 days after the production when kept at a temperature of 25 degrees C during storage.

22. The liquid milk product according to any of the embodiments 11-21 comprising at least 0.20 mmol available NH$_2$-groups per g milk serum protein.

23. The liquid milk product according to any of the embodiments 11-22 wherein the liquid milk product is sterile.

24. A powdered milk product containing the solids of the liquid milk product according to any of the embodiments 11-23.

25. A method of producing a food product, the method comprising the steps of:

i) providing a GOS-containing, liquid milk product according to any of the embodiments 11-23 and/or a GOS-containing, powdered milk product according to embodiment 24,

ii) optionally, providing one or more additional ingredients,

iii) converting the GOS-containing milk product and optionally also the one or more additional ingredient(s) to the foods product, and

iv) optionally, packaging the food product.

26. A food product comprising the GOS-containing, liquid milk product according to any of the embodiments 11-23 and/or the GOS-containing, powdered milk product according to embodiment 24.

27. The food product according to embodiment 26, wherein the food product is one or more of the following:

- an acidified dairy product
- a cheese
- a beverage, and
- a solid, coherent product.

28. Use of a beta-galactosidase enzyme having

- a T-value of at most 0.6, and/or
- a ratio between its transgalactosylation activity and its hydrolase activity of at least 1,

in the production of lactose-reduced milk products to reduce the level of maillard-reaction in the final milk products and/or to extend the shelf-life of the final milk products.

29. Use according to embodiment 28 wherein the milk product is a UHT treated, low lactose milk or lactose-free milk.

**EXAMPLES**

**Example 1 Production of liquid milk products according to the present invention**

3 samples of 10 mL pasteurized skimmed milk containing 5% (w/w) lactose were heated to 4, 6 and 8 degrees C and mixed with 10 microL enzyme solution containing BIF917 (BIF917 and its preparation is described in WO 2013/182686).
Sub-samples of 1 mL were taken after 20 and 24 hours of incubation with the enzyme.
Carrez reagents 1 & 2 were added to the sub-samples in sufficient amounts to cause coagulation of all the solids. The subsequent biphasic mixture was filtered using standard filter paper and once again using a disposable PTFE micro-filter 0.45 microM. At this point, the clear solution was subjected to heat to denature any remaining protein, ion exchange solid phase extraction. The final clear solutions of saccharides were then placed in HPLC vials and analysed.

The HPLC method used was as follows:

System: Agilent
Column Agilent HiPlex Na polymeric ion exchange column
Eluent: MilliQ water

Column: Temperature: 85 degrees C
Flow rate: 0.2 mL/min
Pressure: 21bar (max 25 for this column)
Detector: RID at 35 degrees C

Quantification was based on peak area and known lactose concentration. Response factors for all saccharides were also calculated. Retention times of oligosaccharides have previously been determined using analytical standards of monosaccharides and DP2, 3, and 4 oligosaccharides from Carbosynth UK.

Results:

The sub-samples all contained a substantial amount of GOS and it was clear that the BIF917 retained its transgalactosylation activity despite the low temperature of the incubations.

Surprisingly, the GOS yield in all samples was higher than 1.0% (w/w) (and higher than 20% (w/w) relative to the total amount of carbohydrate) and the total amount of monosaccharides was lower than 2.2% (w/w) (43% (w/w) relative to the total amount of carbohydrate).

The results relating to the sample which was incubated at 8 degrees C are shown in Figure 1. It can be seen that the GOS yield in the 24 h sub-sample is approx. 1.4% (w/w) (approx. 27% (w/w) relative to the total amount of carbohydrate) and that the total amount of monosaccharides were approx. 2.1% (w/w) (42% (w/w) relative to the total amount of carbohydrate).

The detailed carbohydrate compositions of the samples after 20 h and 24 h are shown in the table below:

Table 1 Characterisation of the carbohydrate composition of the 20 h milk sample and the 24 h milk sample

| Carbohydrate species | 20 h sample | 24 h sample |
|---|---|---|
| DP1 - total (g/100 g) | 2.02 | 2.11 |
| Glucose (g/100 g) | 1.61 | 1.64 |
| Galactose (g/100 g) | 0.413 | 0.469 |
| DP2 - total (g/100 g) | 1.62 | 1.53 |
| Lactose (g/100 g) | 0.3 | 0.3 |
| GOS-DP2 species (g/100 g) | 1.30 | 1.23 |
| DP3 (g/100 g) | 0.899 | 0.930 |
| DP4 (g/100 g) | 0.316 | 0.310 |
| DP5 (g/100 g) | 0.098 | 0.091 |
| DP6 (g/100 g) | 0.030 | 0.027 |
| DP7 (g/100 g) | Detectable but not quantified | Detectable but not quantified |
| GOS-DP3+ (g/100 g) | 1.35 | 1.36 |
| **Parameters** | | |
| Glucose/Galactose weight ratio | 3.9 | 3.5 |
| GOS-DP4+/GOS-DP3 weight ratio | 0.49 | 0.46 |
| GOS-DP5/GOS-DP3 weight | 0.11 | 0.10 |

It is worth noting that the milk contained a surprisingly high GOS content and a high content of GOS-DP3+ fraction considering that the milk had been produced by synthesis at 8 degrees C. The ratio between glucose and galactose was furthermore surprisingly high.

The present inventors have observed that the lactose level can be reduced even further by extending the duration of the incubation with the enzyme while maintaining a total content of GOS-DP3+ > 1.0% (w/w) and a weight ratio between glucose and galactose of at least 3:1. They have furthermore observed that incubation at lower temperature such as 4 degrees C also provide a liquid milk product having the same attractive characteristics as that obtained from incubation at 8 degrees C.

The sensory properties of the milk samples prepared as described above have been evaluated and have been reported to taste more milk-like and less sweet than lactose-reduced milk based on fully hydrolysis of lactose.

The inventors have also seen indications that the product resulting from the above process provides a product with a better long-term stability, e.g. due to the more favourable carbohydrate profile and due to the fact that enzymatic processing has been performed at at most 10 degrees C instead of 40-45 degrees C which is perceived as the normal range in the prior art.

**Example 2 Production of a UHT-treated liquid milk product or a powdered milk product according to the present invention**

100 kg of pasteurized skimmed milk (5% lactose, 0.1 % milk fat) is heated to 8 degrees C in a temperature controlled tank and mixed with 100 g of the same enzyme solution containing BIF917 which was used in Example 1. The mixture is allowed to incubate in a storage tank for 24 hours under strict temperature control and is then subjected to heat-treatment (95 degrees C for 10 minutes) in order to inactivate the BIF917-enzyme. The obtained GOS-containing milk is subsequently cooled to 5 degrees C.

50 kg of the GOS-containing milk is converted to a GOS-containing milk powder by standard spray-drying using an inlet air temperature of 180 degrees C. The GOS-containing milk powder contains approx. 4% (w/w) water.

50 kg of the GOS-containing milk is UHT-treated at 143 degrees C for 2 seconds and subsequently filled aseptically in 1 L flasks.

Packaged samples of both the UHT-treated GOS-containing milk and the GOS-containing milk powder are stored at 25 degrees and tested with respect to chemical stability and changes in sensory properties.

**Example 3 Comparison to the prior art**

The results obtained in Example 1 have been comparable to those obtained by Rodrigues-Colinas 2014 and the comparative data is presented in Table 2.

Table 2 Detailed carbohydrate compositions of the milk samples of Examples 1 after 20 h and 24 h compared with the GOS-containing milk sample obtained in Rodriguez-Colinas 2014 (concentration data have been converted from the g/mL to g/100 g assuming a milk density of 1.03 g/mL).

| Carbohydrate species | 20 h sample | 24 h sample | Rodriguez-Colinas 2014 (4 degrees C, table 2) |
|---|---|---|---|
| DP1 - total (g/100 g) | 2.02 | 2.11 | 1.22 |
| Glucose (g/100 g) | 1.61 | 1.64 | 0.79 |
| Galactose (g/100 g) | 0.413 | 0.469 | 0.44 |
| DP2 - total (g/100 g) | 1.62 | 1.53 | 2.8 |
| Lactose (g/100 g) | 0.32 | 0.30 | 2.4 |
| GOS-DP2 species (g/100 g) | 1.30 | 1.23 | 0.39 |
| DP3 (g/100 g) | 0.899 | 0.930 | 0.67 |
| DP4 (g/100 g) | 0.316 | 0.310 | 0.078 |
| DP5 (g/100 g) | 0.098 | 0.091 | 0 |
| DP6 (g/100 g) | 0.030 | 0.027 | 0 |
| DP7 (g/100 g) | Detectable but not quantified | Detectable but not quantified | 0 |
| GOS-DP3+ (g/100 g) | 1.35 | 1.36 | 0.75 |
| **Parameters** | | | |
| Glucose/Galactose weight ratio | 3.9 | 3.5 | 1.8 |
| GOS-DP4+/GOS-DP3 weight ratio | 0.49 | 0.46 | 0.12 |

(continued)

| Parameters | | | |
|---|---|---|---|
| GOS-DP5/GOS-DP3 weight | 0.11 | 0.10 | No DP5 |

Rodriguez-Colinas et al. obtained a maximum yield of GOS (incl. GOS-DP2) of 0.8% (w/w) corresponding to approx. 15% of the total carbohydrates and obtained it and still had 2.4% (w/w) lactose left in the milk sample which therefore does not qualify as a low lactose milk. It should be noted that if Rodriguez-Colinas et al. would attempt to reduce the lactose levels of the milk characterised in Table 2 (Biolactase, 4 degrees C) by further enzymatic action the GOS concentration would be reduced and the content of monosaccharides would increase significantly.

It is furthermore worth noting that the Glc/Gal-ratio of Rodriguez-Colinas 2014 is 1.8, ie. clearly less than 2:1. Additionally, the milk samples of Rodriguez-Colinas 2014 only contain traces of GOS-DP4 and no GOS-DP5 or GOS-DP6.

**Example 4 Determination of the T-value of a beta-galactosidase enzyme**

The T-value of a beta-galactosidase enzyme is determined according to the assay and formula given below.

Assay:

Prepare 3.3 mL enzyme solution consisting of the beta-galactosidase enzyme to be tested, 10 mM sodium citrate, 1 mM magnesium citrate, 1 mM calcium-citrate, Milli-Q water (Millipore, USA) and having a pH of 6.5. The enzyme solution should contain the beta-galactosidase enzyme in an amount sufficient to use 33% (w/w) of the added lactose in 1 hour under the present assay condition. The temperature of the enzyme solution should be 37 degrees C.

At time = $T_0$ 82.5 mg lactose monohydrate (for biochemistry, Merck Germany) is added to and mixed with the enzyme solution, and the mixture is subsequently incubated at 37 degrees C for 4 hours. Precisely 1 hour after $T_0$ a 100 microL sample is collected and is diluted 1:5 with Milli-Q water and inactivated by heating to 95 degrees C for 10 min. The inactivated mixture is kept at -20 degrees C until the characterization.

Characterisation:

The determination of the amount (in mol) of produced galactose and the amount of used lactose (in mol) may be performed using any suitable analysis technique. For example, the diluted mixture may be analyzed by HPLC according to the method described by Richmond et al. (Separation of Carbohydrates in Dairy Products by High Performance Liquid Chromatography, J. of Dairy Science, 65 (8), 1394-1400, 1982) and Simms et al. (Separations of lactose, lactobionic and lactobionolactose by high performance liquid chromatography. J. of Chromatography, 667, 67-73., 1994).

Another example of a suitable analysis technique is ISO 5765-2:2002 (IDF 79-2: 2002) "Dried milk, dried ice-mixes and processed cheese - Determination of lactose content - Part 2: Enzymatic method utilizing the galactose moiety of the lactose".

Calculation of the T-value:

The T-value is calculated according to the following formula using the data obtained from the characterization of the diluted mixture of the assay:

$$\text{T-value} = \frac{\text{amount of produced galactose (in mol)}}{\text{amount of used lactose (in mol)}}$$

Example - T-value of the BIF917 enzyme:

The above-mentioned assay was performed using the BIF917 enzyme of WO 2013/182686.

The diluted mixture obtained from the assay was analyzed with respect to converted (i.e. used) lactose and generated galactose via analytical HPLC. The HPLC apparatus was from Waters and equipped with a differential refractometer (RI-detector) and a BioRad Aminex HPX-87C column (300x7.8 mm, 125-0055). Elution of saccharides was performed isocratically with 0.05 g/L CaAcetate, a flow rate of 0.3 mL/min. and an injection volume of 20 microL.

The obtained data was appropriately baseline corrected by automated software, peaks were individually identified and integrated. Quantification was performed by using external standards of lactose monohydrate (for biochemistry, Merck, Germany), D-(+)-glucose monohydrate (for biochemistry, Merck Eurolab, France), and D-(+)-galactose (≥99%, Sigma-Aldrich, Italy).

The conversion of lactose and the formation of galactose to each time T was calculated from the quantified data. At time T=1h 29% of the lactose in the collected 100 microL sample had been converted, which corresponds to 2.3 micromol lactose. At time T=1 0.5 micromol galactose had been formed in the collected 100 microL sample. The T-value can therefore be calculated to 0.5 micromol / 2.3 micromol = 0.2.

The diluted mixture obtained from the assay was also analyzed with respect to converted (i.e. used) lactose and generated galactose via the enzymatic method ISO 5765-2. A Boehringer Mannheim Lactose/D-Galactose test-kit from R-Biopharm (Cat. No. 10 176 303 035) was used and the test was performed according to the protocol. The enzymatic method confirmed a T-value of the BIF917-enzyme of 0.2.

Example - T-value of conventional lactase enzyme:

The above-mentioned assay was performed using the commercially available conventional lactase enzyme Lactozym Pure 2600L (Novozymes, Denmark). The diluted mixture obtained from the assay was analyzed as described for the BIF917 enzyme. Tri- and tetra-saccharides were not present in detectable amounts and equal amounts of glucose and galactose were observed. The corresponding T-value is 1.

The T-values of commercially available beta-galactosidase from Escherichia coli (Product number: G6008, Sigma-Aldrich, Germany) and Aspergillus oryzae (Product number: G5160, Sigma-Aldrich, Germany) have also been determined, and both enzymes have a T-value of approx. 1.0.

## Claims

1. A liquid milk product having a pH of at least 5.0 comprising

   - at least 0.9% (w/w) GOS,
   - at most 1.5% (w/w) lactose,

- a total content of monosaccharide of at most 3.0% (w/w), and
- a weight ratio between glucose and galactose of least 2:1.

2. The liquid milk product according to claim 1 comprising at least 1.0% (w/w) GOS.

3. The liquid milk product according to claim 1 or 2 comprising at most 1.0% (w/w) lactose.

4. The liquid milk product according to any of the claims 1-3 having a total content of monosaccharide of at most 2.0% (w/w).

5. The liquid milk product according to any of the claims 1-4 having a total amount of glucose in the range of 0.8-2.0% (w/w) and a total amount of galactose in the range of 0.2-0.6% (w/w).

6. The liquid milk product according to any of the claims 1-5 having a weight ratio between glucose and galactose of least 3:1.

7. The liquid milk product according to any of the claims 1-6 comprising inactivated beta-galactosidase enzyme.

8. The liquid milk product according to any of the claims 1-7 comprising a total amount of GOS-DP3+ of at least 0.5% (w/w).

9. The liquid milk product according to any of the claims 1-8 wherein the weight ratio between GOS-DP4+ and GOS-DP3 is at least 0.15.

10. The liquid milk product according to any of the claims 1-9 wherein the weight ratio between GOS-DP5 and GOS-DP3 is at least 0.01.

11. The liquid milk product according to any of the claims 1-10 having a furosine value of at most 60 mg/100 g protein 7 days after the production when kept at a temperature of 25 degrees C during storage.

12. The liquid milk product according to any of the claims 1-11 comprising at least 0.20 mmol available $NH_2$-groups per g milk serum protein.

13. The liquid milk product according to any of the claims 1-12 wherein the liquid milk product is sterile.

14. A powdered milk product containing the solids of the liquid milk product according to any of the claims 1-13.

15. A method of producing a food product, the method comprising the steps of:

    i) providing a GOS-containing, liquid milk product according to any of the claims 1-13 and/or a GOS-containing, powdered milk product according to claim 14,
    ii) optionally, providing one or more additional ingredients,
    iii) converting the GOS-containing milk product and optionally also the one or more additional ingredient(s) to the foods product, and
    iv) optionally, packaging the food product.

16. A food product comprising the GOS-containing, liquid milk product according to any of the claims 1-13 and/or the GOS-containing, powdered milk product according to claim 14.

17. The food product according to claim 16, wherein the food product is one or more of the following:

    - an acidified dairy product
    - a cheese
    - a beverage, and
    - a solid, coherent product.

18. Use of a beta-galactosidase enzyme having

- a T-value of at most 0.6, and/or
- a ratio between its transgalactosylation activity and its hydrolase activity of at least 1,

in the production of lactose-reduced milk products to reduce the level of maillard-reaction in the final milk products and/or to extend the shelf-life of the final milk products.

19. Use according to claim 18 wherein the milk product is a UHT treated, low lactose milk or lactose-free milk.

# Fig. 1

Europäisches
Patentamt

European
Patent Office

Office européen
des brevets

# EUROPEAN SEARCH REPORT

Application Number

EP 19 16 8718

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 272 095 A2 (YAKULT HONSHA KK [JP]) 22 June 1988 (1988-06-22) | 1-5,7, 11-17 | INV. A23C9/12 A23C9/13 |
| Y | * page 4, line 16 - line 17 * <br> * page 5, line 6 - line 32; claims 1-6; examples 1,2,3 * | 6,8-10 | A23C9/18 A23C21/02 C12P19/04 |
| A | EP 2 526 784 A1 (NESTEC SA [CH]) 28 November 2012 (2012-11-28) * paragraphs [0028] - [0033], [0055] - [0102]; claims 1-22; examples 1-3 * | 1-19 | C12N9/10 C12N9/38 A23C9/16 C12P19/14 |
| X,D | WO 2013/182686 A1 (DUPONT NUTRITION BIOSCI APS [DK]) 12 December 2013 (2013-12-12) | 18,19 | |
| Y | * page 2, line 25 - page 3, line 11 * <br> * page 7, line 33 - page 8, line 16 * <br> * page 20, line 8 - line 11 * <br> * page 22, line 23 - line 25 * <br> * page 24, line 28 - line 34 * <br> * examples 1-4 * <br> * page 11, line 26 - page 12, line 2 * | 6,8-10 | |
| X,D | WO 2011/120993 A1 (DANISCO [DK]; LARSEN MORTEN KROG [DK]; POULSEN CHARLOTTE HORSMANS [DK]) 6 October 2011 (2011-10-06) | 18 | TECHNICAL FIELDS SEARCHED (IPC) A23C C12P |
| Y | * example 3 * | 6,8-10 | C12N |

The present search report has been drawn up for all claims

| Place of search | Date of completion of the search | Examiner |
|---|---|---|
| Munich | 29 July 2019 | Schlegel, Birgit |

EPO FORM 1503 03.82 (P04C01)

## EP 3 542 633 A1

### ANNEX TO THE EUROPEAN SEARCH REPORT
### ON EUROPEAN PATENT APPLICATION NO.

EP 19 16 8718

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2019

| Patent document cited in search report | | Publication date | Patent family member(s) | | Publication date |
|---|---|---|---|---|---|
| EP 0272095 | A2 | 22-06-1988 | AU | 603165 B2 | 08-11-1990 |
| | | | CA | 1333779 C | 03-01-1995 |
| | | | EP | 0272095 A2 | 22-06-1988 |
| | | | KR | 920007687 B1 | 14-09-1992 |
| | | | NZ | 222902 A | 28-08-1990 |
| EP 2526784 | A1 | 28-11-2012 | AU | 2012260945 A1 | 31-10-2013 |
| | | | BR | 112013029700 A2 | 17-01-2017 |
| | | | CA | 2839797 A1 | 29-11-2012 |
| | | | CL | 2013003359 A1 | 11-07-2014 |
| | | | CN | 103547173 A | 29-01-2014 |
| | | | EP | 2526784 A1 | 28-11-2012 |
| | | | EP | 2713775 A1 | 09-04-2014 |
| | | | MX | 344054 B | 02-12-2016 |
| | | | RU | 2013157161 A | 27-06-2015 |
| | | | SG | 194645 A1 | 30-12-2013 |
| | | | US | 2014087021 A1 | 27-03-2014 |
| | | | US | 2016278421 A1 | 29-09-2016 |
| | | | WO | 2012160080 A1 | 29-11-2012 |
| | | | ZA | 201309667 B | 27-09-2017 |
| WO 2013182686 | A1 | 12-12-2013 | AU | 2013273507 A1 | 18-12-2014 |
| | | | BR | 112014030009 A2 | 12-09-2017 |
| | | | CN | 104334720 A | 04-02-2015 |
| | | | CO | 7240398 A2 | 17-04-2015 |
| | | | DK | 2859098 T3 | 11-12-2017 |
| | | | EA | 201492221 A1 | 30-04-2015 |
| | | | EP | 2859098 A1 | 15-04-2015 |
| | | | EP | 3305896 A1 | 11-04-2018 |
| | | | EP | 3473712 A1 | 24-04-2019 |
| | | | EP | 3473713 A1 | 24-04-2019 |
| | | | EP | 3473714 A1 | 24-04-2019 |
| | | | ES | 2650444 T3 | 18-01-2018 |
| | | | HK | 1207665 A1 | 05-02-2016 |
| | | | JP | 2015518729 A | 06-07-2015 |
| | | | JP | 2018148905 A | 27-09-2018 |
| | | | NZ | 702287 A | 24-06-2016 |
| | | | PL | 2859098 T3 | 28-02-2018 |
| | | | US | 2015223481 A1 | 13-08-2015 |
| | | | WO | 2013182686 A1 | 12-12-2013 |
| | | | ZA | 201408880 B | 23-12-2015 |
| WO 2011120993 | A1 | 06-10-2011 | DK | 2552945 T3 | 16-07-2018 |
| | | | EP | 2552945 A1 | 06-02-2013 |
| | | | EP | 3392268 A1 | 24-10-2018 |
| | | | ES | 2681692 T3 | 14-09-2018 |

EPO FORM P0459

For more details about this annex : see Official Journal of the European Patent Office, No. 12/82

page 1 of 2

**ANNEX TO THE EUROPEAN SEARCH REPORT**
**ON EUROPEAN PATENT APPLICATION NO.**                    EP 19 16 8718

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2019

| Patent document cited in search report | Publication date | Patent family member(s) | Publication date |
|---|---|---|---|
| | | US 2013059034 A1 | 07-03-2013 |
| | | US 2015307861 A1 | 29-10-2015 |
| | | WO 2011120993 A1 | 06-10-2011 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013182686 A
- WO 200190317 A2
- WO 2013182686 A1
- WO 2011120993 A1
- WO 2012010699 A1

**Non-patent literature cited in the description**

- **MACFARLANE et al.** *Aliment Pharmacol Ther,* vol. 24, 701-714
- **RODRIGUES-COLINAS et al.** *J. Agric. Food Chem.,* 2012, vol. 60, 6391-6398
- **RODRIGUES-COLINAS et al.** *Food Chemistry,* 2014, vol. 145, 388-394
- **F. CHEVALIER et al.** *International Dairy Journal,* 2001, vol. 11, 145-152
- **RICHMOND et al.** Separation of Carbohydrates in Dairy Products by High Performance Liquid Chromatography. *J. of Dairy Science,* 1982, vol. 65 (8), 1394-1400
- **SIMMS et al.** Separations of lactose, lactobionic and lactobionolactose by high performance liquid chromatography. *J. of Chromatography,* 1994, vol. 667, 67-73